(19) **Europäisches Patentamt / European Patent Office / Office européen des brevets**

(11) **EP 1 936 522 A1**

(12) **EUROPÄISCHE PATENTANMELDUNG**

(43) Veröffentlichungstag:
   **25.06.2008   Patentblatt 2008/26**

(51) Int Cl.:
   ***G06F 19/00*** *(2006.01)*

(21) Anmeldenummer: **06026806.7**

(22) Anmeldetag: **22.12.2006**

(84) Benannte Vertragsstaaten:
   **AT BE BG CH CY CZ DE DK EE ES FI FR GB GR HU IE IS IT LI LT LU LV MC NL PL PT RO SE SI SK TR**
   Benannte Erstreckungsstaaten:
   **AL BA HR MK RS**

(71) Anmelder: **InterComponentWare AG**
   **69190 Walldorf (DE)**

(72) Erfinder: **Heinze, Peter, Dr.**
   **69190 Walldorf (DE)**

(74) Vertreter: **Müller-Boré & Partner**
   **Patentanwälte**
   **Grafinger Strasse 2**
   **81671 München (DE)**

(54) **Automatische Priorisierung medizinischer Betreuungen**

(57)   Um zumindest einen Patienten und/oder eine Gruppe von Patienten automatische zu priorisieren, um dadurch medizinische Betreuungen von Patienten zu optimieren, ist ein Verfahren und ein System zu einer zu einer automatischen Priorisierung von medizischen Betreuungen von zumindest einem Patienten vorgesehen. Das Verfahren umfaßt die Schritte:

- Erfassen von einem oder mehreren Werten des zumindest einen Patienten;

- Bestimmen zumindest eines Trends des zumindest einen Patienten zu einer entsprechenden Größe;

- Bereitstellen eines Satzes von gewichteten Funktionen $p_1,...,p_m$ und eines Satzes von medizinischen Größen $t_1,...,t_m$, wobei jeweils zumindest eine gewichtete Funktion $p_i$ des Satzes von gewichteten Funktionen jeweils zumindest einer Größe $t_i$ des Satzes von medizinischen Größen zugeordnet ist;

- Anwenden des Satzes von gewichteten Funktionen auf einen oder mehrere Werte des zumindest einen Patienten und/oder auf zumindest einen Trend des zumindest einen Patienten, wobei jeweils zumindest eine gewichtete Funktion $p_i$ des Satzes von gewichteten Funktionen auf zumindest einen der zumindest einen zugeordneten Größe $t_i$ entsprechenden Wert $x_i$ und/oder auf zumindest einen der zumindest einen zugeordneten Größe $t_i$ entsprechenden Trend $x_i$ angewendet wird;

- Addieren und Normalisieren der jeweiligen gewichteten Funktionen zum Erhalt einer Priorisierung; und

- Ausgeben einer Betreuungsreihenfolge für den zumindest einen Patienten, welche der Priorisierung entspricht.

FIG 3

EP 1 936 522 A1

**Beschreibung**

Die vorliegende Erfindung bezieht sich generell auf medizinische Betreuungen von Patienten. Spezifischer bezieht sich die vorliegende Erfindung auf ein computer-implementiertes Verfahren, ein System und ein Computerprogrammprodukt zur automatischen Priorisierung von medizinischen Betreuungen von Patienten.

Angesichts der beschränkten Ressourcen im Gesundheitssystem, insbesondere Überwachung, Betreuung und Vorsorge chronisch kranker und alter Patienten, bietet Telemedizin Möglichkeiten, die bekannten Formen der Betreuung und Überwachung von Patienten zu ergänzen und darüber hinaus diese mit relativ geringem Aufwand auf Patienten- und Betreuungsseite zu intensivieren. Telemedizinische Betreuung ergänzt einen traditionellen, zeit- und personalintensiven Hausbesuch bzw. den regelmäßigen Praxisbesuch durch eine klar strukturierte, reproduzierbare und auf moderner Technik basierende Kommunikation zwischen Arzt und Patient. So kann eine andernfalls weder zu leistende noch zu finanzierende hohe Frequenz von Patientenkontakten erfolgen.

In bekannten Systemen zur telemedizinischen Betreuung hat jeder Patient mindestens einen Sensor, mit dem täglich mindestens ein Wert erfaßt und an ein telemedizinisches Zentrum zur Befundung übermittelt wird. In dem telemedizinischen Zentrum werten Ärzte und/oder ausgebildete Krankenschwestern erfaßte Werte und deren zeitliche Entwicklung im Kontext erhobener Gesundheitsdaten zumindest eines Patienten aus. In den meisten bekannten Systemen zur medizinischen Betreuung werden insbesondere ein oder mehrere Werte hinsichtlich einer speziellen Erkrankung, wie beispielsweise Asthma, Herzinsuffizienz oder Diabetes Mellitus überwacht. Sollen nun aber mehrere Werte und deren zeitliche Entwicklung erfaßt, übermittelt und in einem telemedizinischen Zentrum ausgewertet werden, so wird es wichtig, Patienten ein optimale Betreuung zu ermöglichen. Insbesondere kann es erforderlich sein, die im telemedizinischen Zentrum auflaufenden Betreuungsaufgaben in eine medizinisch sinnvolle zeitliche Bearbeitungsreihenfolge zu bringen.

Über eine optimale telemedizinische Betreuung hinaus, kann es auch notwendig sein, in einem Krankenhaus anfallende Betreunungsaufgaben eines oder mehrerer Patienten in eine medizinisch sinnvolle zeitliche Reihenfolge zu bringen.

Aufgabe der Erfindung ist es, medizinische Betreuungen von Patienten computergestützt zu optimieren.

Diese Aufgabe ist entsprechend der Erfindung durch die Merkmale der unabhängigen Ansprüche gelöst. Bevorzugte Ausführungsformen der Erfindung sind Gegenstand der abhängigen Ansprüche.

Entsprechend der Erfindung ist ein computer-implementiertes Verfahren zu einer automatischen Priorisierung von medizischen Betreuungen von zumindest einem Patienten vorgesehen, die Schritte umfassend:

- Erfassen von einem oder mehreren Werten des zumindest einen Patienten;
- Bestimmen zumindest eines Trends des zumindest einen Patienten zu einer entsprechenden Größe;
- Bereitstellen eines Satzes von gewichteten Funktionen und eines Satzes von medizinischen Größen, wobei jeweils zumindest eine gewichtete Funktion des Satzes von gewichteten Funktionen jeweils zumindest einer Größe des Satzes von medizinischen Größen zugeordnet ist;
- Anwenden des Satzes von gewichteten Funktionen auf einen oder mehrere Werte des zumindest einen Patienten und/oder auf zumindest einen Trend des zumindest einen Patienten, wobei jeweils zumindest eine gewichtete Funktion des Satzes von gewichteten Funktionen auf zumindest einen der zumindest einen zugeordneten Größe entsprechenden Wert und/oder auf zumindest einen der zumindest einen zugeordneten Größe entsprechenden Trend angewendet wird;
- Addieren und Normalisieren der jeweiligen gewichteten Funktionen zum Erhalt einer Priorisierung; und
- Ausgeben einer Betreuungsreihenfolge für den zumindest einen Patienten, welche der Priorisierung entspricht.

Entsprechend ist ein computer-implementiertes Verfahren zu einer automatischen Priorisierung von medizischen Betreuungen von zumindest einem Patient und/oder einer Gruppe von Patienten vorgesehen. Bevorzugt umfaßt das computerimplementierte Verfahren einen Satz an Eingabewerten, welcher durch einen oder mehrere einer oder mehrerer medizinischer Meßgrößen entsprechenden ermittelten medizinischen Werten eines oder mehreren Patienten und/oder einer oder mehrerer Trends über einen oder mehrere einer Größe entsprechenden erfaßten Werten, welche bevorzugt über ein gegebenes Zeitintervall erfaßt und zumindest einem der einen oder mehreren Patienten zugeordnet wurden, ermittelt wird, und einen Ausgabewert. Der Ausgabewert stellt eine Priorisierung einer Berteuungsaufgabe zumindest eines Patienten dar und ermöglicht es somit, einen optimalen Vorschlag für eine Bearbeitungsreihenfolge des zumindest einen Patienten und/oder einer Gruppe von Patienten in einer medizinischen Betreuungseinrichtung (insbesondere ein medizinischen Zentrum und/oder ein telemedizinischen Zentrum) zu ermitteln.

Entsprechend bevorzugt beschreibt ein Trend eine Relativveränderung von einem oder mehreren einer Größe entsprechenden Werten des zumindest einen Patienten innerhalb eines vorgegebenen Zeitraums. Entsprechend ist ein Trend zumindest ähnlich zu einem oder mehren erfaßten Werten ein Absolutwert, welcher in eine Bestimmung bzw. Berechnung einer Priorisierung einfließt. Weiterhin können zumindest teilweise Veränderungen der erfaßten Werte und/oder Trends des zumindest einen Patienten bestimmt werden.

Weiterhin bevorzugt werden die erfaßten Werte des zumindest einen Patienten in einer Datenverwaltungseinrichtung (insbesondere einer elektronischen Patientenakte) dem zumindest einen Patienten zugeordnet und gespeichert. Weiterhin umfassen die erfaßten Werte insbesondere Werte einer entsprechenden medizinischen Größe und/oder Diagnosewerte für den zumindest einen Patienten.

Weiterhin kann bevorzugt zusätzlich zu der Priorisierung eine Beschreibung ausgegeben werden, welche die ermittelte Priorisierung durch einen Kommentar ergänzt und/oder begründet.

Bevorzugt umfaßt der Schritt des Erfassens von einem oder mehreren Werten zumindest eines Patient die Schritte:

- Erfassen eines Wertes eines Körpergewichts des zumindest einen Patienten; und/oder
- Erfassen eines Wertes einer Herzfrequenz des zumindest einen Patienten; und/oder
- Erfassen eines Wertes eines systolischen Blutdrucks des zumindest einen Patienten; und/oder
- Erfassen einer zuvor manuell gesetzten Priorisierung des zumindest einen Patienten; und/oder
- Erfassen eines Wertes einer Dauer einer PQ-Strecke des zumindest einen Patienten; und/oder
- Erfassen eines Wertes einer Dauer einer QRS-Strecke des zumindest einen Patienten; und/oder
- Erfassen eines Wertes einer Dauer einer QTc-Strecke des zumindest einen Patienten; und/oder
- Erfassen einer Selbsteinschätzung des zumindest einen Patienten; und/oder
- Erfassen eines in einem EKG erfaßten Vorhofflimmers des zumindest einen Patienten; und/oder
- Erfassen eines in einem EKG erfaßten AV Blockes ersten, zweiten oder dritten Grades des zumindest einen Patienten; und/oder
- Erfassen eines in einem EKG erfaßten ventrikuläre Tachykardie des zumindest einen Patienten.

Weiterhin bevorzugt erfolgt der Schritt des Erfassens von einem oder mehreren Werten des zumindest einen Patienten durch zumindest ein geeignetes medizinisches Gerät.

Entsprechend können medizinische Geräte zur Erfassung von einem oder mehreren Werten eines oder mehreren Patienten, insbesondere eine Körperwaage und/oder ein Blutdruckmeßgerät und/oder ein Gerät zum Erfassen eines Elektrokardiogramms (EKG) verwendet werden. Ein EKG ist eine Erfassung einer Menge ein oder mehrerer elektrischer Aktivitäten von Herzmuskelfasern eines Herzens zumindest eines Patienten.

Weiterhin bevorzugt erfolgt der Schritt des Erfassens von einem oder mehreren Werten des zumindest einen Patienten durch zumindest einen Assessmentbogen.

Entsprechend kann der zumindest eine Patient zumindest einen Wert insbesondere über Selbsteinschätzungen zu seinem Gesundheitszustand erheben, welche in einer Priorisierung berücksichtigt werden.

Weiterhin bevorzugt umfaßt der Schritt des Bestimmens zumindest eines Trends des zumindest einen Patienten zu einer entsprechenden Größe den Schritt:

- Erfassen von zumindest einem einer Größe entsprechenden Wert innerhalb eines Zeitintervalls.

Bevorzugter beträgt das Zeitintervall 24 Stunden und/oder 8 Tagen.

Am meisten bevorzugt ist die Größe ein Körpergewicht und/oder eine Herzfrequenz und/oder ein systolischer Blutdruck des zumindest einen Patienten.

Entsprechend kann ein Trend (insbesondere eine Relativveränderung) eines Körpergewichts und/oder einer Herzfrequenz und/oder eines systolischen Blutdrucks des zumindest einen Patienten in den letzten 24 Stunden und/oder in den letzten 8 Stunden erfaßt werden und entsprechend als zumindest einer der Eingabewerte des Verfahren zu einer automatischen Priorisierung von medizischen Betreuungen von zumindest einem Patient dienen.

In einem weiteren Aspekt der vorliegenden Erfindung umfaßt der Schritt des Bestimmen zumindest eines Trends des zumindest einen Patienten zu einer entsprechenden Größe die Schritte:

- Berechnen des Trends durch eine Steigung einer Geraden durch zwei Punkte, wobei die zwei Punkte zumindest zwei Werte der Menge von erfaßten Werten innerhalb des Zeitintervalls umfaßt; und/oder
- Berechnen des Trends durch eine Steigung einer Geraden, welche in einem "Least-Square-Fitting" Verfahren in die Menge von Werten gelegt wird, welche innerhalb des Zeitintervalls erfaßt sind.

Weiterhin bevorzugt umfaßt der schritt des Addierens und Normalisierens der jeweiligen gewichteten Funktionen ein Normalisieren auf einen Wertebereich [0..1].

Entsprechend weist der Ausgabewert, welcher die Priorisierung der Betreuungsaufgabe des zumindest einen Patienten bestimmt, einen Wert zwischen 0 und 1 auf. Eine Priorität 1 eines Ausgabewertes ist hoch, eine Priorität 0 eines Ausgabewertes ist niedrig.

Entsprechend der Erfindung ist weiterhin ein System zu einer automatischen Priorisierung von medizinischen Betreuungen von zumindest einem Patienten gemäß Anspruch 11 vorgesehen.

Ein weiterer Aspekt der vorliegenden Erfindung betrifft ein Computerprogrammprodukt, insbesondere auf einem computerlesbaren Medium gespeichert oder als Signal verwirklicht, welches, wenn geladen in den Speicher eines Computers und ausgeführt von einem Computer, bewirkt, daß der Computer ein erfindungsgemäßes Verfahren oder eine bevorzugte Ausführungsform hiervon durchführt.

Diese und andere Objekte, Merkmale und Vorteile der vorliegenden Erfindung werden durch Lesen der folgenden detaillierten Beschreibung von bevorzugten Ausführungsformen und begleitenden Zeichnungen offensichtlicher werden. Es sollte so zu verstehen sein, daß selbst wenn Ausführungsformen separat beschrieben sind, einzelne Merkmale davon zu zusätzlichen Ausführungsformen kombiniert werden können.

Figur 1 zeigt eine Verarbeitungskette von Werten eines zumindest einen Patienten.

Figur 2 zeigt ein System zu einer automatischen Priorisierung von medizinischen Betreuungen von zumindest einem Patienten.

Figur 3 zeigt einen schematischen Ablauf eines Verfahrens zu einer automatischen Priorisierung von medizinischen Betreuungen.

Figur 3A zeigt eine schematische Darstellung eines Satzes von Eingabewerte und einen Ausgabewert eines Verfahrens zu einer automatischen Priorisierung von medizinischen Betreuungen.

Figur 4 zeigt eine Basisfunktion zu einem Eingabewert.

Figur 5 zeigt eine Basisfunktion zu einer Veränderung eines Körpergewichtes über 24 Stunden.

Figur 6 zeigt eine Basisfunktion zu einer Veränderung eines Körpergewichtes über 8 Tage.

Figur 7 zeigt eine Basisfunktion zu einer Veränderung einer Herzfrequenz über 24 Stunden.

Figur 8 zeigt eine Basisfunktion zu einer Veränderung einer Herzfrequenz über 8 Tage.

Figur 9 zeigt eine Basisfunktion zu einer Veränderung eines systolischen Blutdrucks über 24 Stunden.

Figur 10 zeigt eine Basisfunktion zu einer Veränderung eines systolischen Blutdrucks über 8 Tage.

Figur 11 zeigt eine Basisfunktion zu einer letzten Priorisierung.

Figur 12 zeigt eine Basisfunktion zu einer EKG PQ Dauer.

Figur 13 zeigt eine Basisfunktion zu einer EKG QRS Dauer.

Figur 14 zeigt eine Basisfunktion zu einem Vorhofflimmern.

Figur 15 zeigt eine Basisfunktion zu einem AV-Block.

Figur 16 zeigt eine Basisfunktion zu einer ventrikulären Tachykardie.

Figur 17 zeigt eine Basisfunktion zu einer Selbsteinschätzung.

Figur 18 zeigt ein Blockdiagramm eines technische Aufbaus eines Computers und eines Netzwerkes.

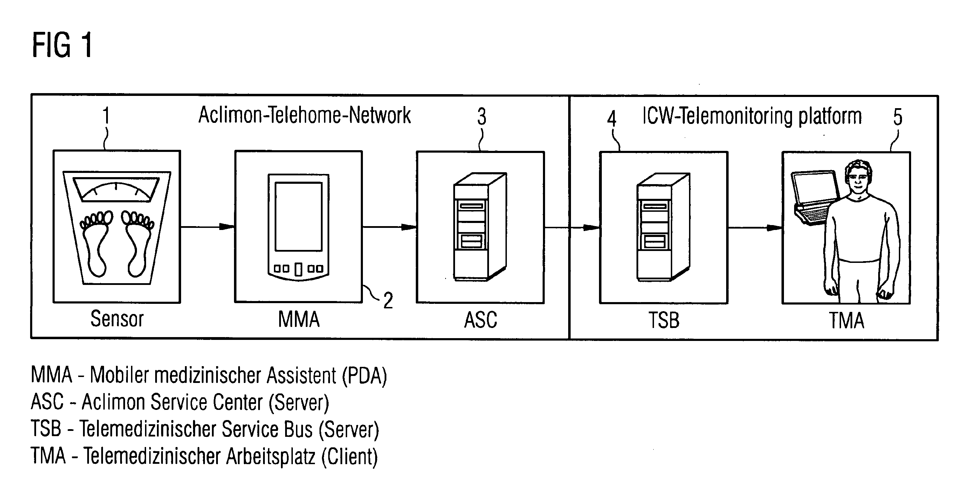

Bezugnehmend auf Figur 1 wird eine bevorzugte Ausführungsform eines Systems und Verfahrens zur automatischen, computergestützten Verarbeitung eines oder mehrerer Werte von zumindest einem Patient und/oder einer Gruppe von Patienten gezeigt. Ein beispielhaftes System und Verfahren zur Verarbeitung von Werten umfaßt zumindest einen Sensor 1, zumindest einen mobilen Assistenten 2 (insbesondere ein mobiler medizinischer Assistent), einen ersten Server 3, einen zweiten Server 4 und einen Client 5, wobei der erste Server 3 bevorzugt ein Service Center und am meisten bevorzugt ein Actimon Service Center (der Firma Actimon), der zweite Server 4 bevorzugt ein Service Bus und am meisten bevorzugt ein telemedizinischer Service Bus und der Client 5 bevorzugt ein telemedizinischer Arbeitsplatz ist. Gemäß Figur 1 werden mittels des zumindest einen Senors 1 ein oder mehrere Werte des zumindest einen Patienten und/oder der Gruppe von Patienten, welche einer oder mehreren Größen entsprechen, erfaßt. Die ein oder mehreren erfaßten Werte werden drahtlos (beispielsweise mittels Bluetooth) bevorzugt in verschlüsselter Form an den zumindest einen mobilen medizinischen Assistenten 2 übertragen. Weiterhin bevorzugt werden die ein oder mehreren erfaßten Werte (automatisch) und bevorzugt in verschlüsselter Form von dem zumindest einem mobilen medizinischen Assistenten 2 an den Service Center 3 übertragen. Der Service Center 3 überträgt automatisch und bevorzugt in verschlüsselter Form die ein oder mehreren erfaßten Werte an den Service Bus 4. Weiterhin bevorzugt werden der eine oder die mehreren erfaßten Werte bei einer Übertrag von dem Service Center 3 an den Service Bus 4 aufbereitet. In einer Datenverwaltungseinrichtung des Service Busses 4 werden die ein oder mehreren Werte mit einem Zeitstempel ergänzt, dem zumindest einen Patienten zugeordnet und gespeichert. Der Service Bus 4 erzeugt anhand der einen oder mehreren empfangenen Werte des zumindest einen Patienten und/oder zumindest eines Trends über einen oder mehrere einer Größe entsprechenden Werte des zumindest einen Patienten und/oder der Gruppe von Patienten, wobei die ein oder mehreren einer Größe entsprechenden Werte (zumindest teilweise) innerhalb eines gegebenen Zeitintervalls erfaßt werden, eine priorisierte Bearbeitungsaufgabe, welche anschließend in dem telemedizinischen Arbeitsplatz 5 zur Verfügung gestellt wird.

Entsprechend kann der telemedizinischen Arbeitsplatz eine graphische Benutzeroberfläche aufweisen, welche zu jedem Patienten eine Priorität, eine Berarbeitungsaufgabe und eine der Priorität entsprechende Rangnummer in einer Bearbeitungsreihenfolge anzeigt. Weiterhin kann die graphische Benutzeroberfläche einen Zugriff auf die von einem Patienten erfaßten und gespeicherten Werte ermöglichen. Neben der Priorität kann eine Beschreibung angegeben sein, welche zusammen mit der Priorität in der graphischen Benutzeroberfläche angezeigt wird. In der Beschreibung kann die Priorität für den zumindest einen Patienten (zumindest teilweise) in textueller Form erläutert und/oder begründet sein. Bevorzugte Ausgabewerte einer Priorität und einer Beschreibung sind in Tabelle 1 aufgeführt.

**Tabelle1**

| ID | Name | Einheit | Beschreibung |
|----|------|---------|--------------|
| 1 | Priority | [0..1] | Priorität auf Basis gemessener Werte und/oder ermittelter Trends im Wertebereich zwischen 0 und 1. Die Priorität 1 ist hoch, die Priorität 0 ist niedrig |
| 2 | Description | String | Text, welcher die berechnete Priorität begründet. |

Entsprechend eines oder mehreren erfaßten Werten und/oder Trends über einen oder mehrere erfaßte Werte zumindest eines Patienten wird eine Bearbeitungsaufgabe und eine Priorität für den zumindest einen Patienten ermittelt. Eine Priorität ist ein Wert zwischen 0 und 1, wobei 0 eine niedrige und 1 eine hohe Priorität bezeichnet. Entsprechenden ist die Bearbeitungsaufgabe priorisiert. Mittels der Priorität kann eine Nummer bzw. Rangnummer für den zumindest einen Patienten in einer Bearbeitungsreihenfolge eines oder mehrerer Patienten bestimmt werden. Beispielsweise hat ein Patient A eine Priorität 0,61, ein Patient B eine Priorität 0,54 und ein Patient C eine Priorität 0,71. Entsprechend der Prioritäten kann für die Patienten A, B, und C folgende Bearbeitungsreihenfolge ermittelt und in dem telemedizinischen Arbeitsplatz beispielsweise über eine graphische Benutzeroberfläche angezeigt werden: 1. C , 2. A, 3. B, weil die Priorität von C (0,71) größer ist als die von A (0,61), welche wiederum größer ist als die Priorität von B (0,54).

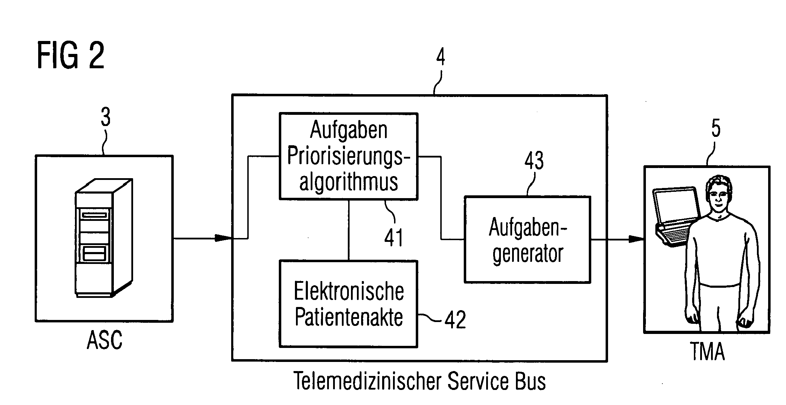

Wie in Figur 2 in einer bevorzugten Ausführungsform gezeigt, werden die ein oder mehreren empfangenen Werte des zumindest einen Patienten und/oder einer Gruppe von Patienten innerhalb des Service Busses 4 verarbeitet. Dazu werden die ein oder mehreren empfangenen Werte an ein Priorisierungsverfahren 41, welches in dem Service Bus 4 implementiert ist, übergeben. Weiterhin bevorzugt werden die einen oder mehreren empfangenen Werte des zumindest einen Patient und/oder einer Gruppe von Patienten dem zumindest einen Patienten und/oder einem jeweiligen Patienten der Gruppe von Patienten zugeordnet und durch einen Zeitstempel ergänzt, welcher einen Zeitpunkt angibt, wann die ein oder mehreren Werte erfaßt wurden. Die erfaßten Werte werden bevorzugt zusammen mit dem entsprechenden erfaßten Zeitstempel dem entsprechenden Patienten zugeordnet und in einer elektronischen Patientenakte 42 gespeichert. Das Priorisierungsverfahren 41 ermittelt anhand von einer Menge von Werten und/oder Trends über einen oder mehrere erfaßte Werte einer entsprechenden Größe innerhalb eines Zeitintervalls des zumindest einen Patienten und/oder der Gruppe von Patienten einen Wert für eine Priorität, mit welcher der zumindest eine Patient und/oder ein jeweiliger Patient der Gruppe von Patienten in einem medizinischen Zentrum von einem Arzt und/oder einer entsprechend ausgebildeten Krankenschwester durch den telemedizinischen Arbeitsplatz 5 oder einen medizinischen Arbeitsplatz beispielsweise in einem Krankenhaus zu behandeln ist.

Der Wert für eine Priorität wird ermittelt und zwar auf Basis der ein oder mehreren empfangenen Werte und/oder Trends des zumindest einen Patienten und/oder der Gruppe von Patienten und/oder eines oder mehreren älteren Werten des zumindest einen Patienten und/oder einer Gruppe von Patienten, welche bevorzugt innerhalb eines gegebenen Zeitintervalls erfaßt wurden. Nachdem ein Wert für eine Priorität ermittelt wurde, wird eine Betreuungsaufgabe durch einen Aufgabengenerator 43 in dem telemedizinischen Arbeitsplatz 5 ermittelt und anschließend erzeugt, dessen Bearbeitungsreihenfolge durch den Wert für die Priorität bestimmt wird.

Die elektronische Patientenakte 42 umfaßt eine Datenbank, welche zu dem zumindest einen Patienten und/ oder einem jeweiligen Patienten der Gruppe von Patienten medizinische Informationen und Daten erfaßt und speichert, welche übergreifend und transparent abgerufen werden können. Weiterhin erfaßt die elektronische Patientenakte 42 Dokumente (beispielsweise zumindest eine Diagnose), welche den zumindest einen Patienten und/oder jeweils einen Patienten der Gruppe von Patienten betreffen und welche als Verweise in einer Liste gesammelt werden.

Ein (medizinisches) Notfallereignis zumindest eines Patienten wird explizit durch das telemedizinische Zentrum ausgelöst, wenn ein solches Notfallereignis von dem zumindest einen Patienten entweder automatisch über ein im Wesentlichen dem in Figur 1 gezeigten entsprechenden System und/oder durch eine direkte Angabe des zumindest einen Patienten in dem telemedizinischen Zentrum ausgelöst wurde. Für ein solches Notfallereignis wird keine Wert für eine Priorität ermittelt und keine Aufgabenbetreuung erzeugt. Entsprechend wird ein solches Notfallereignis ad-hoc abgearbeitet und ist damit nicht Bestandteil einer Bearbeitungsreihenfolge, d.h., ein solches Notfallereignis wird nicht in eine Warteschlange der Berabeitungsreihenfolge eingereiht.

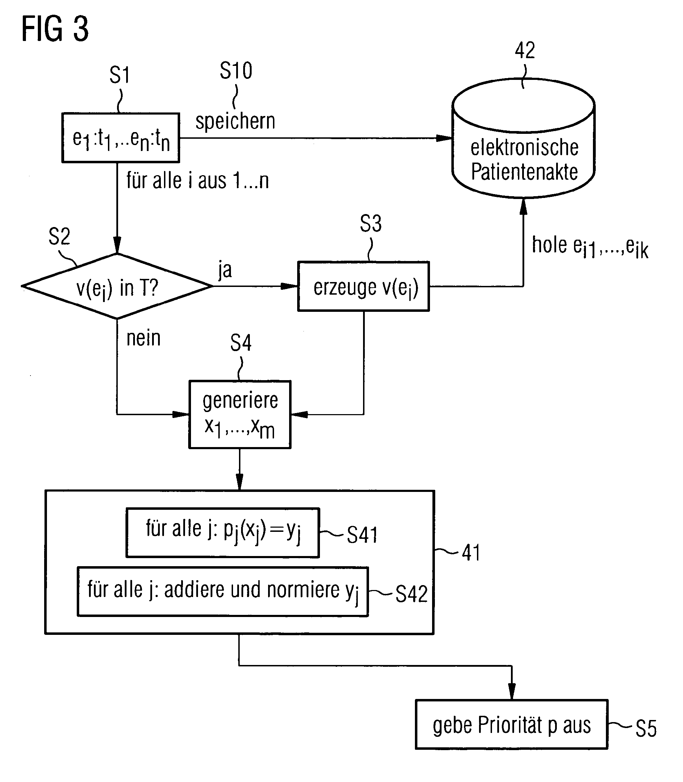

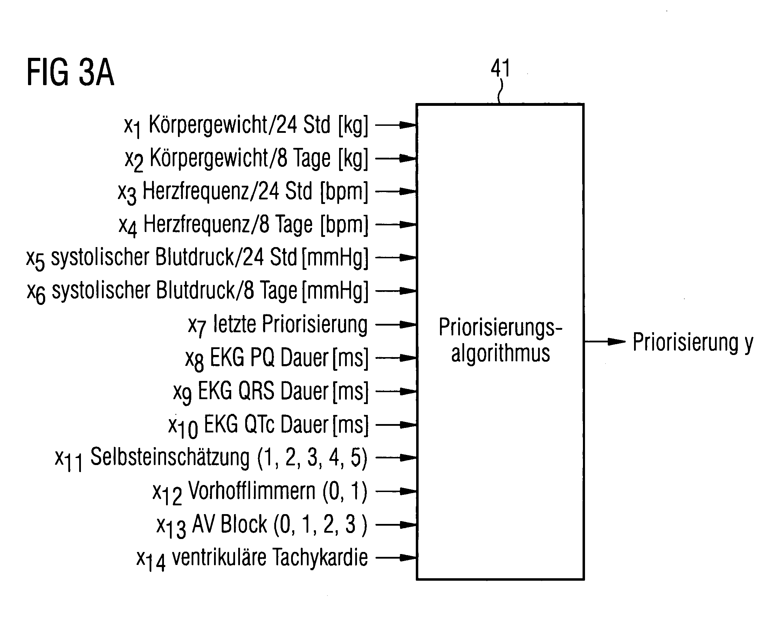

Figur 3 zeigt eine bevorzugte Ausführungsform des Priorisierungsverfahrens 41. Zumindest ein einem Satz von Größen $t_1,...,t_n$ entsprechender Satz von Werten $e_1,...,e_n$ zumindest eines Patienten P wird erfaßt (S1). Der Satz von Werten wird in der elektronischen Patientenakte 42 dem zumindest einen Patienten zugeordnet, mit einem Zeitstempel ergänzt und gespeichert (10). Für jede Größe $t_i$ des Satzes von Größen wird geprüft, ob ein Trend $v(t_i)$ zu bestimmen ist (S2). Um einen Trend zu einer Größe $t_i$ zu bestimmen, werden ein oder mehrere Werte $e_{i1},...,e_{ik}$ der entsprechenden Größe $t_i$ des zumindest einen Patienten P aus der elektronischen Patientenakte 42 geladen bzw. ermittelt, welche innerhalb eines gegebenen Zeitintervalls T, bevorzugt innerhalb der letzten 24 Stunden und/oder innerhalb der letzten 8 Tage, erfaßt wurden. Entsprechend wird ein Trend (insbesondere eine Relativveränderung) über die Werten $e_{i1},...,e_{ik}$ innerhalb des gegebenen Zeitintervalls T ermittelt (S3). Ein bevorzugtes Verfahren zur Bestimmung eines Trends ist weiter unten ausführlicher beschrieben.

Aus dem Satz von erfaßten Werten $e_1,...,e_n$ und/oder einer oder mehreren erzeugten Trends von Werten einer Größe $t_i$ innerhalb des Zeitintervalls T wird ein Satz von Eingabewerten $x_1,...,x_m$ generiert (S4). Der Satz von Eingabewerten $x_1,...,x_m$ dient als Eingabe für das Priorisierungsverfahren 41. Innerhalb des Priorisierungsverfahrens 42 wird auf jeden Eingabewert $x_j$ des Satzes von Eingabewerten $x_1,...,x_m$ eine spezifische Funktion $p_j$ eines Satzes von Priorisierungsfunktionen $p_1,...,p_m$ mit $p_j(x_j) = y_j$ angewendet (S41), wobei $y_i$ eine Priorität darstellt. Weiterhin ist jede Funktion des Satzes von Priorisierungsfunktionen $p_1,...,p_m$ gewichtet. Eine bevorzugte Ausführungsform des Satzes von Funktionen $p_1,...,p_m$ ist weiter unten ausführlich beschrieben. Die Ergebnisse $y_1,...,y_m$ werden addiert und normalisiert (S42), um eine Priorität y zu erhalten und auszugeben (S5). Folglich weist die Priorität einen Wertebereich zwischen 0 und 1 auf, wobei eine Priorität 0 niedrig und eine Priorität 1 hoch ist.

Ein bevorzugter Satz von Eingabewerten $x_1,...,x_m$ für das Priorisierungsverfahren 41 ist in Figur 3A gezeigt. Entsprechend umfaßt ein Satz von erfaßten Werten $e_1,...,e_n$ zumindest eines Patienten ein oder mehrere Werte, welche einem Körpergewicht, einer Herzfrequenz, einem systolischen Blutdruck, einer letzten manuell gesetzten Priorisierung, einer Dauer einer PQ-Strecke, einer Dauer einer QRS-Strecke, einer Dauer einer QTc-Strecke, einer Selbsteinschätzung, einem in einem EKG erfaßtes Vorhofflimmern, einem in einem EKG erfaßten AV-Block und/oder einem in einem EKG erfaßten ventrikularen Tachykardie des zumindest einen Patienten und/oder der Gruppe von Patienten entsprechen. Weiterhin wird ein Trend eines Körpergewichtes innerhalb der letzten 24 Stunden und innerhalb der letzten 8 Tage, ein Trend einer Herzfrequenz innerhalb der letzten 24 Stunden und/oder innerhalb der letzten 8 Tage und/oder ein Trend eines systolischen Blutdrucks innerhalb der letzten 24 Stunden und innerhalb der letzten 8 Tage des zumindest einen Patienten P bestimmt und zusammen mit den restlichen der oben genannten Werte als Eingabewerte $x_1,...,x_m$ für eine Bestimmung einer Priorisierung y durch das Priorisierungsverfahren 41 verwendet, wie es in Figur 3A gezeigt ist. Entsprechend zeigt die Tabelle 1 einen einem Satz von Größen $t_1,...,t_m$ entsprechenden Satz von Eingabewerte $x_1,...,x_m$ zur Bestimmung einer Priorisierung y des zumindest einen Patienten.

Eine Kurve eines EKGs läßt sich in mehrere Abschnitte einteilen, welchen jeweils bestimmten elektrophysioligischen Vorgängen in einem Herz eines Patienten zugrunde liegen. Entsprechend ist eine PQ-Strecke im Wesentlichen eine isoelektrische, d.h. horizontal verlaufende Linie, welche von einem Ende einer P-Welle bis zu einem Anfang eines QRS-Komplexes bzw. einer QRS-Strecke reicht. Die PQ-Strecke entspricht der Zeit von einem Ende einer Vorhoferregung bis zu einem Anfang einer Kammereregung. Die P-Welle entsteht durch eine Ausbreitung einer Erregung in den Vorhöfen eines Herzens. Der QRS-Komplex ist ein scharf gezackter Komplex, welcher einer Depolarisation beider Kammern entspricht. Eine QTc-Strecke bzw. QTc-Dauer schließ im wesentlichen eine QRS-Komplex, eine ST-Strecke und eine T-Welle ein. Die Dauer einer QTc-Strecke entspricht einer Kammersystole und ist abhängig von einer Herzfrequenz. Die ST-Strecke ist eine isoelektrische Linie vom Ende des QRS-Komplexes bis zum Anfang der T-Welle. Die T-Welle entsteht durch eine Erregungsrückbildung der Herzkammern.

Entsprechend ist ein AV-Block (atrioventrikulärer Block) eine Herzrhythmusstörung, wobei die Erregungsleitung zwischen den Vorhöfen und den Herzkammern verzögert, zeitweise oder dauerhaft unterbrochen ist. Bei einem AV-Block ersten Grades ist die Erregungsleitung verzögert. Folglich kommt es zu einer verspätet einsetzenden Kontraktion der Herzkammern. Ein AV-Block ersten Grades ist an einer Verlängerung einer PQ-Dauer (über 200 ms) in einem EKG erkennbar. Bei einem AV-Block zweiten Grades fällt die Erregungsleitung teilweise aus. Ein AV-Block dritten Grades ist dadurch gekennzeichnet, daß die Erregungsleitung zwischen Vorhof und Kammer vollständig ausfällt.

**Tabelle 2**

| Name | Größe $t_i$ | Beschreibung |
|---|---|---|
| Körpergewicht / 24h | kg | Veränderung des Körpergewichts in den letzten 24 Stunden p(200 g/d) = 0,5 |
| Körpergewicht / 8d | kg | Veränderung des Körpergewichts in den letzten 8 Tagen; F(500/g/8d)= 0,5; 24h zu 8d gleichgewichtet |
| Herzfrequenz / 24h | bpm (beats per minute) | Veränderung der Herzfrequenz in den letzten 24 Stunden (vermutlich EKG) F(+15)=0.5 ; F(-i 5)=075 |
| Herzfrequenz / 8d | bpm | Veränderung der Herzfrequenz in den letzten 8 Tagen F (÷15)=0 5 F(-15)=0 75; 24h zu 8d gleichgewichtet |
| systolischer Blutdruck / 24h | mmHg | Veränderung des systolischen Blutdrucks in den letzten 24 Stunden F(+35) = 0.5; F(- 20)=0,5 |
| systolischer Blutdruck / 8d | mmHg | Veränderung des systolischen Blutdrucks in den letzten 8 Tagen F(+35) = 05; F(- 20)=05 24h zu 8d gleichberechtigt |
| letzte Priorisierung | - | eine in der letzten Befundung manuell vergebene Priorisierung. 0 ist niedrig, 1 ist hoch |
| EKG PQ Dauer | ms | Dauer der PQ-Strecke |
| EKG ORS Dauer | ms | Dauer der QRS-Strecke |
| EKG QTc Dauer | ms | Dauer der QTc-Strecke |
| Vorhofflimmern | 0,1 | Ein im EKG detektiertes Vorhofflimmern |
| AV- Block | 1,2,3 | Ein im EKG detektierter AV Block L,2, oder 3. Grades |
| ventrikuläre Tachykardie | 0,1 | Eine im EKG detektierte ventrikuläre Tachykardie |
| Selbsteinschätzung | 1,2,3,4,5 | Die letzte übertragene Selbsteinschätzung des Patienten auf einer Skala von 1-5.. 1 steht für gut, 5 für schlecht. |

Bevorzugt wird bei einem Körpergewicht, einer Herzfrequenz und/oder einem systolischen Blutdruck des zumindest einen Patienten ein Trend über einer Göße entsprechenden Werten ermittelt, welche in der elektronischen Patientenakte 42 dem zumindest einen Patienten zugeordnet und gespeichert sind und zumindest teilweise innerhalb eines Zeitintervalls erfaßt wurden. Sofern kein Wert innerhalb des Zeitintervalls vorhanden ist, können keine Trends berechnet werden. Ein Wert für eine entsprechende Gewichtung, welcher in eine Bestimmung einer Priorität eingeht, wird in einem solchen Fall auf 0 gesetzt. Entsprechend werden in einem gegebenen Zeitintervall eine Menge von Werten eines Körpergewichts, einer Herzfrequenz und/oder eines systolischen Blutdrucks des zumindest einen Patienten erfaßt und in der elektronischen Patientenakte 42 gespeichert. Bevorzugt ist das gegebene Zeitintervall eine Zeitspanne von 24 Stunden und/oder 8 Tagen. Weiterhin bevorzugt werden für das gegebene Zeitintervall Trends eines Körpergewichts, einer Herzfrequenz und/oder eines systolischen Blutdrucks des zumindest einen Patienten folgendermaßen berechnet:

- Sind zu einem Körpergewicht, einer Herzfrequenz und/oder einem systolischen Blutdruck des zumindest einen Patienten nur 0 oder 1 einer dieser Größen entsprechenden Werte für den zumindest eine Patienten innerhalb des gegebenen Zeitintervalls erfaßt, so kann kein Trend für ein Körpergewicht, eine Herzfrequenz und/oder einen systolischen Blutdruck des zumindest einen Patienten ermittelt werden.
- Sind zu einem Körpergewicht, einer Herzfrequenz und/oder einem systolischen Blutdruck des zumindest einen Patienten ein Wert erfaßt, so kann eine relative Abweichung zu einem der Göße entsprechenden Standardwert, welcher auf den zumindest einen Patient bezogen ist, ermittelt werden. Ein Standardwert könnte sich auf ein durchschnittliches Körpergewicht, eine durchschnittliche Herzfrequenz und/oder einen durchschnittlichen systolischen Blutdruck einer dem zumindest einen Patienten entsprechender Durchschnittsperson entsprechenden Alters und Geschlechts beziehen.

- Sind zu einem Körpergewicht, einer Herzfrequenz und/oder einem systolischen Blutdruck des zumindest einen Patienten zwei einer dieser Größen entsprechende Werte in der für den zumindest eine Patienten innerhalb des gegebenen Zeitintervalls erfaßt, so wird ein Trend über die erfaßten Werte eines Körpergewichts, einer Herzfrequenz und/oder eines systolischen Blutdrucks entsprechend durch eine Steigung einer Geraden durch die zwei Werte, welche durch Punkte in einem Koordinatensystem bezüglich des Zeitintervalls und einer der erfaßten Werte entsprechenden Größe dargestellt werden können, ermittelt.

- Sind zu einem Körpergewicht, einer Herzfrequenz und/oder einem systolischen Blutdruck des zumindest einen Patient mehr als 2 dieser Größen entsprechende Werte für den zumindest eine Patienten innerhalb des gegebenen Zeitintervalls erfaßt, so wird ein Trend über die erfaßten Werte eines Körpergewichts, einer Herzfrequenz und/oder eines systolischen Blutdrucks entsprechend durch eine Steigung einer Geraden ermittelt, welche beispielsweise durch ein "Least-Square-Fitting" Verfahren in die mehr als zwei entsprechenden Werte gelegt wird, welche durch Punkte in einem Koordinatensystem bezüglich des Zeitintervalls und einer der erfaßten Werte entsprechenden Größe dargestellt werden können.

Im folgenden wird eine bevorzugte Ausführung eines beispielhaften Satzes von Funktionen $p_1,...,p_m$ zur Ermittlung einer Priorisierung gezeigt, welche als Eingabewerte einen Satz von Eingabewerten $x_1,...,x_m$ entsprechender Größen $t_1,...,t_m$ erfordert: $p_1(x_1:t_1), ..., p_m(x_m:t_m)$. Entsprechend ist für jeden Eingabewert $x_i$ eines Satzes von Eingabewerten $x_1,...,x_m$ eine spezifische Priorisierungsfunktion $p_i$ bestimmt, wobei ein Eingabewert $x_i$ entweder ein einer Größe $t_i$ entsprechender Wert $e_i$ zumindest eines Patienten ist, welcher erfaßt wurde, oder ein Trend $v(t_i)$ über einer Größe $t_i$ entsprechenden Werten $e_{i1},...,e_{ik}$ des zumindest einen Patienten, welche innerhalb eines gegebenen Zeitintervalls erfaßt wurden. Ein Ergebnis der spezifischen Funktion $p_i(x_i)$ stellt eine Priorität $y_i$ einer aktuellen Erfassung des zumindest einen erfaßten Wertes $e_i$ oder eines Trends $v(t_i)$ einer Größe $t_i$ entsprechenden Werte dar, welche innerhalb eines gegebenen Zeitintervalls erfaßt wurden.

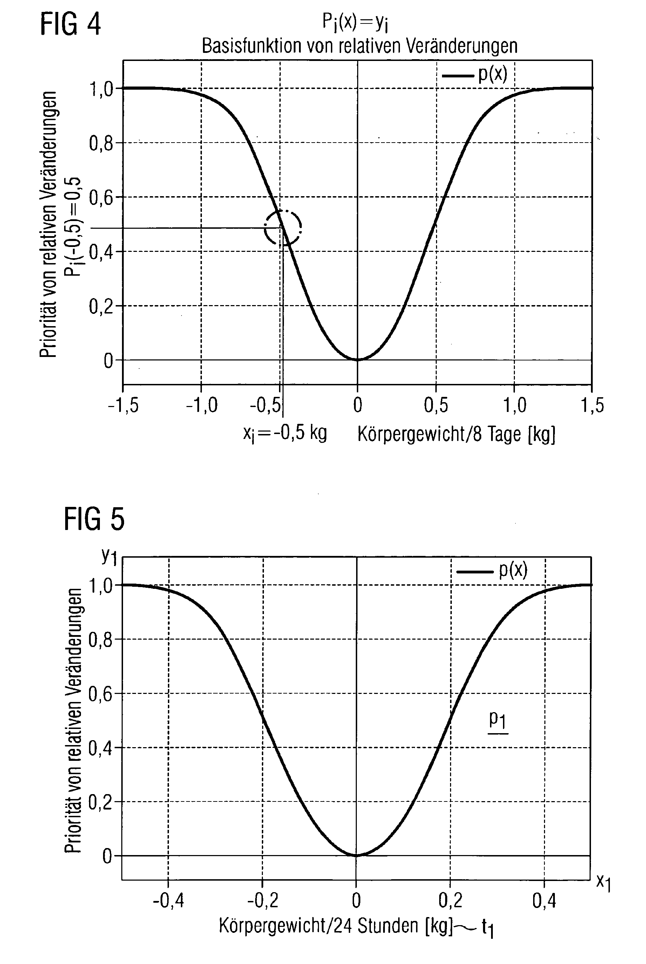

Weiterhin bevorzugt wird für jede einem Eingabewert x; entsprechende Größe $t_i$ eine beispielhafte Basisfunktion für jede Prioritätsfunktion $p_i$ vorgegeben. Eine beispielhafte Basisfunktion für einen Trend eines Körpergewichtes innerhalb eines Zeitintervalls von 8 Tagen zumindest eines Patienten ist in Figur 4 gezeigt. Entsprechend kann beispielsweise bei einer relativen Veränderung von -0,5 kg ($x_i$ = - 0,5 kg) eines Körpergewichts zumindest eines Patienten innerhalb von 8 Tagen eine Priorität von 0,5 ($p_i(-0,5) = 0,5$) der Basisfunktion für einen Trend eines Körpergewichtes innerhalb eines Zeitintervalls von 8 Tagen des zumindest einen Patienten ermittelt werden. Entsprechend einer beispielhaften Basisfunktion für einen Trend eines Körpergewichtes innerhalb eines Zeitintervalls von 8 Tagen kann diese Basisfunktion so interpretiert werden, daß eine geringe Gewichtszunahme innerhalb von 8 Tagen zumindest eines Patienten "unkritisch" ist, und damit eine niedrige Priorität zugewiesen bekommt, wobei dann jede weitere Gewichtszunahme innerhalb von 8 Tagen eine exponentielle Verschlechterung des Gesundheitszustandes bedeuten kann, und somit die Priorität bei entsprechender Gewichtszunahme exponentiell steigt und damit den zumindest einen Patienten in einer Bearbeitungsreihenfolge durch eine relativ höhere Priorität nach vorne schiebt.

In einem weiteren Aspekt der vorliegenden Erfindung weist ein Satz von Basisfunktionen $p_1,...,p_m$ zur Ermittlung einer Priorsierung zumindest eines Patienten aus einem Satz von Eingabewerten $x_1,...,x_m$ entsprechenden Satz von Größen $t_1,...,t_m$ folgende bevorzugte und beispiehafte Ausführungsformen auf.

Eine Basisfunktion zur Ermittlung einer Priorität eines Trends eines Körpergewichts zumindest eines Patienten über 24 Stunden ist beispielsweise und bevorzugt definiert durch:

$$p(x) = \frac{2}{1 + \exp(-x \cdot x \cdot 27,5)} - 1. \qquad (1)$$

Eine der Formel (1) entsprechende graphische Darstellung ist in Figur 5 gezeigt. In Tabelle 3 sind mögliche Prioritäten angegeben, welche durch einen entsprechend ermittelten Trend eines Körpergewichts (in kg) über 24 Stunden des zumindest einen Patienten gemäß der Formel (1) ermittelt werden können.

**Tabelle 3**

| Veränderung in kg | Priorität | Veränderung in kg | Priorität |
|---|---|---|---|
| -0,5 | 0,998 | 0,025 | 0,009 |

(fortgesetzt)

| Veränderung in kg | Priorität | Veränderung in kg | Priorität |
|---|---|---|---|
| -0,4 | 0,976 | 0,05 | 0,034 |
| -0,3 | 0,845 | 0,075 | 0,077 |
| -0,2 | 0,501 | 0,1 | 0,137 |
| -0,15 | 0,300 | 0,15 | 0,300 |
| -0,1 | 0,137 | 0,2 | 0,501 |
| -0,075 | 0,077 | 0,3 | 0,845 |
| -0,05 | 0,034 | 0,4 | 0,976 |
| -0,025 | 0,009 | 0,5 | 0,998 |
| 0.0 | 0,000 | - | - |

Beispielsweise weist entsprechend der Basisfunktion $p_1$ ein Trend von $x_1 = 0,15$ eines Körpergewichts zumindest eines Patienten, welcher innerhalb eines Zeitintervalls von 24 Stunden aus entsprechenden erfaßten Werten eines Körpergewichts des zumindest einen Patienten ermittelt wurde, eine Priorität $y_1 = 0,300$ auf. Damit ist eine geringe Gewichtszunahme des zumindest einen Patienten innerhalb von 24 Stunden als unkritisch zu betrachten und erhält somit eine niedrige Priorität, während eine jede weitere Gewichtszunahme eine exponentielle Verschlechterung bedeuten bzw. entsprechen kann, so daß die entsprechende Priorität entsprechend exponentiell steigt.

Eine Basisfunktion zur Ermittlung einer Priorität eines Trends eines Körpergewichts zumindest eines Patienten über 8 Tage ist bevorzugt definiert durch:

$$p(x) = \frac{2}{1 + \exp(-x \cdot x \cdot 4,4)} - 1. \qquad (2)$$

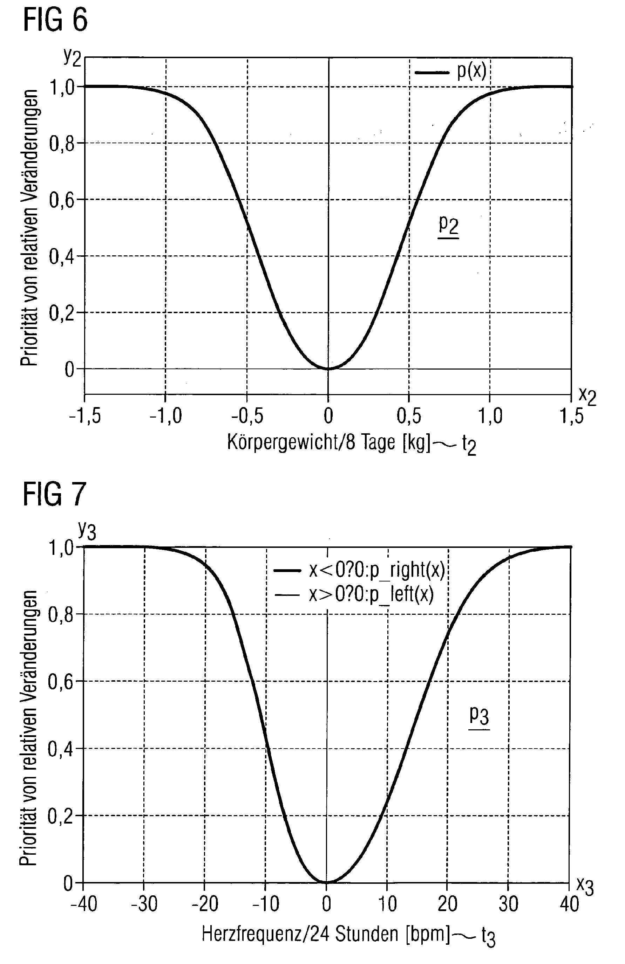

Eine Formel (2) entsprechende graphische Darstellung ist in Figur 6 gezeigt. In einer Tabelle 4 sind mögliche Prioritäten angegeben, welche durch einen entsprechend ermittelten Trend eines Körpergewichts über 8 Tage des zumindest einen Patienten gemäß der Formel (2) ermittelt werden können.

**Tabelle 4**

| Veränderung in kg | Priorität | Veränderung in kg | Priorität |
|---|---|---|---|
| -2,000 | 1,000 | 0,100 | 0,022 |
| -1,500 | 1,000 | 0,200 | 0,088 |
| -1,000 | 0,976 | 0,300 | 0,195 |
| -0,500 | 0,501 | 0,400 | 0,338 |
| -0,400 | 0,338 | 0,500 | 0,501 |
| -0,300 | 0,195 | 1,000 | 0,976 |
| -0,200 | 0,088 | 1,500 | 1,000 |
| -0,100 | 0,022 | 2,000 | 1,000 |
| 0,00 | 0,00 | - | - |

Beispielsweise weist entsprechend der Basisfunktion $p_2$ ein Trend von $x_2 = 0,500$ eines Körpergewichts zumindest eines Patienten, welcher innerhalb eines Zeitintervalls von 8 Tagen aus entsprechenden erfaßten Werten eines Körpergewichts des zumindest einen Patienten ermittelt wurde, eine Priorität $y_2 = 0,501$ auf. Damit ist eine geringe Gewichtszunahme des zumindest einen Patienten innerhalb von 8 Tagen als unkritisch zu betrachten und erhält somit eine niedrige Priorität, während eine jede weitere Gewichtszunahme eine exponentielle Verschlechterung bedeuten bzw. entsprechen kann, so daß die entsprechende Priorität entsprechend exponentiell steigt.

Eine Basisfunktion zur Ermittlung einer Priorität eines Trends einer Herzfrequenz zumindest eines Patienten über 24 Stunden ist bevorzugt definiert durch:

$$(x < 0): p\_left(x) = \frac{2}{1 + \exp(-x \cdot x \cdot 0{,}008655)} - 1$$

$$(x > 0): p\_right(x) = \frac{2}{1 + \exp(-x \cdot x \cdot 0{,}004885)} - 1. \qquad (3)$$

Eine Formel (3) entsprechende graphische Darstellung ist in Figur 7 gezeigt. In Tabelle 5 sind mögliche Prioritäten angegeben, welche durch einen entsprechend ermittelten Trend einer Herzfrequenz (in beates per minute (bpm)) über 24 Stunden des zumindest einen Patienten gemäß der Formel (3) ermittelt werden können.

**Tabelle 5**

| Veränderung in bpm | Priorität | Veränderung in bpm | Priorität |
|---|---|---|---|
| -2,000 | 1,000 | 0,100 | 0,022 |
| -1,500 | 1,000 | 0,200 | 0,088 |
| -1,000 | 0,976 | 0,300 | 0,195 |
| -0,500 | 0,501 | 0,400 | 0,338 |
| -0,400 | 0,338 | 0,500 | 0,501 |
| -0,300 | 0,195 | 1,000 | 0,976 |
| -0,200 | 0,088 | 1,500 | 1,000 |
| -0,100 | 0,022 | 2,000 | 1,000 |
| 0,000 | 0,000 | - | - |

Beispielsweise weist entsprechend der Basisfunktion $p_3$ ein Trend von $x_3 = 4000$ einer Herzfrequenz zumindest eines Patienten, welcher innerhalb eines Zeitintervalls von 24 Stunden aus entsprechenden erfaßten Werten einer Herzfrequenz des zumindest einen Patienten ermittelt wurde, eine Priorität $y_3 = 0{,}039$ auf. Damit ist eine geringer Anstieg einer Herzfrequenz des zumindest einen Patienten innerhalb von 24 Stunden als unkritisch zu betrachten und erhält somit eine niedrige Priorität, während ein jeder weiterer Anstieg einer Herzfrequenz eine exponentielle Verschlechterung bedeuten bzw. entsprechen kann, so daß die entsprechende Priorität entsprechend exponentiell steigt.

Eine Basisfunktion zur Ermittlung einer Priorität eines Trends einer Herzfrequenz zumindest eines Patienten über 8 Tage ist bevorzugt definiert durch:

$$(x < 0): p\_left(x) = \frac{2}{1 + \exp(-x \cdot x \cdot 0{,}008655)} - 1$$

$$(x > 0): p\_right(x) = \frac{2}{1 + \exp(-x \cdot x \cdot 0{,}004885)} - 1. \qquad (4)$$

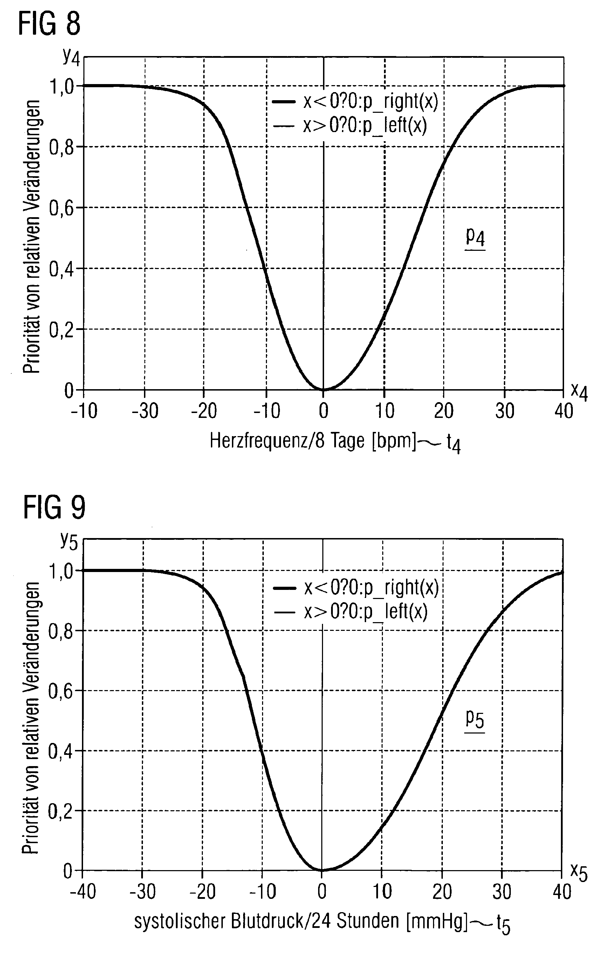

Eine Formel (4) entsprechende graphische Darstellung ist in Figur 8 gezeigt. In Tabelle 6 sind mögliche Prioritäten angegeben, welche durch einen entsprechend ermittelten Trend einer Herzfrequenz über 24 Stunden des zumindest einen Patienten gemäß der Formel (4) ermittelt werden können.

**Tabelle 6**

| Veränderung in bpm | Priorität | Veränderung in bpm | Priorität |
|---|---|---|---|
| -40,000 | 1,000 | 1,000 | 0,002 |
| -30,000 | 0,999 | 2,000 | 0,010 |
| -20,000 | 0,939 | 3,000 | 0022 |
| -1 5,000 | 0,750 | 4,000 | 0,039 |
| -1 0,000 | 0,408 | 5,000 | 0,061 |
| -5,000 | 0,108 | 10,000 | 0,240 |
| -4,000 | 0,069 | 15,000 | 0,500 |
| -3,000 | 0,039 | 20,000 | 0,752 |
| -2,000 | 0,017 | 30,000 | 0,976 |
| -1,000 | 0,004 | 40,000 | 0,999 |
| 0,000 | 0,000 | - | - |

Beispielsweise weist entsprechend der Basisfunktion $p_4$ ein Trend von $x_4 = 4000$ einer Herzfrequenz zumindest eines Patienten, welcher innerhalb eines Zeitintervalls von 8 Tagen aus entsprechenden erfaßten Werten einer Herzfrequenz des zumindest einen Patienten ermittelt wurde, eine Priorität $y_4 = 0,039$ auf. Damit ist eine geringer Anstieg einer Herzfrequenz des zumindest einen Patienten innerhalb von 8 Tagen als unkritisch zu betrachten und erhält somit eine niedrige Priorität, während ein jeder weiterer Anstieg einer Herzfrequenz eine exponentielle Verschlechterung bedeuten bzw. entsprechen kann, so daß die entsprechende Priorität entsprechend exponentiell steigt.

Eine Basisfunktion zur Ermittlung einer Priorität eines Trends eines systolischen Blutdrucks zumindest eines Patienten über 24 Stunden ist bevorzugt definiert durch:

$$(x < 0) : p\_left(x) = \frac{2}{1 + \exp(-x \cdot x \cdot 0{,}008655)} - 1$$

$$(x > 0) : p\_right(x) = \frac{2}{1 + \exp(-x \cdot x \cdot 0.00275)} - 1 \, . \qquad (5)$$

Eine Formel (5) entsprechende graphische Darstellung ist in Figur 9 gezeigt. In Tabelle 7 sind mögliche Prioritäten angegeben, welche durch einen entsprechend ermittelten Trend eines systolischen Blutdrucks (in mmHg) über 24 Stunden des zumindest einen Patienten gemäß der Formel (5) ermittelt werden können.

**Tabelle 7**

| Veränderung in mmHg | Priorität | Veränderung in mmHg | Priorität |
|---|---|---|---|
| -40,000 | 1,000 | 1,000 | 0,001 |
| -30,000 | 0,999 | 2,000 | 0,005 |
| -20,000 | 0,939 | 3,000 | 0,012 |
| -15,000 | 0,750 | 4,000 | 0,022 |
| -10,000 | 0,408 | 5,000 | 0,034 |
| -5,000 | 0,108 | 10,000 | 0,137 |
| -4,000 | 0,069 | 15,000 | 0,300 |
| -3,000 | 0,039 | 20,000 | 0,501 |

(fortgesetzt)

| Veränderung in mmHg | Priorität | Veränderung in mmHg | Priorität |
|---|---|---|---|
| -2,000 | 0,017 | 30,000 | 0,845 |
| -1,000 | 0,004 | 40,000 | 0,976 |
| 0,000 | 0,000 | - | - |

Beispielsweise weist entsprechend der Basisfunktion $p_5$ ein Trend von $x_5 = 4000$ eines systolischen Blutdrucks zumindest eines Patienten, welche innerhalb eines Zeitintervalls von 24 Stunden aus entsprechenden erfaßten Werten eines systolischen Blutdrucks des zumindest einen Patienten ermittelt wurde, eine Priorität $y_5 = 0,022$ auf. Damit ist eine geringer Anstieg eines systolischen Blutdrucks des zumindest einen Patienten innerhalb von 24 Stunden als unkritisch zu betrachten und erhält somit eine niedrige Priorität, während ein jeder weiterer Anstieg eines systolischen Blutdrucks eine exponentielle Verschlechterung bedeutet bzw. entsprechen kann, so daß die entsprechende Priorität entsprechend exponentiell steigt.

Eine Basisfunktion zur Ermittlung einer Priorität eines Trends eines systolischen Blutdrucks zumindest eines Patienten über 8 Tage ist bevorzugt definiert durch:

$$(x < 0)\, p\_left(x) = \frac{2}{1 + \exp(-x \cdot x \cdot 0,008655)} - 1$$

$$(x > 0):\, p\_right(x) = \frac{2}{1 + \exp(-x \cdot x \cdot 0,275)} - 1. \tag{6}$$

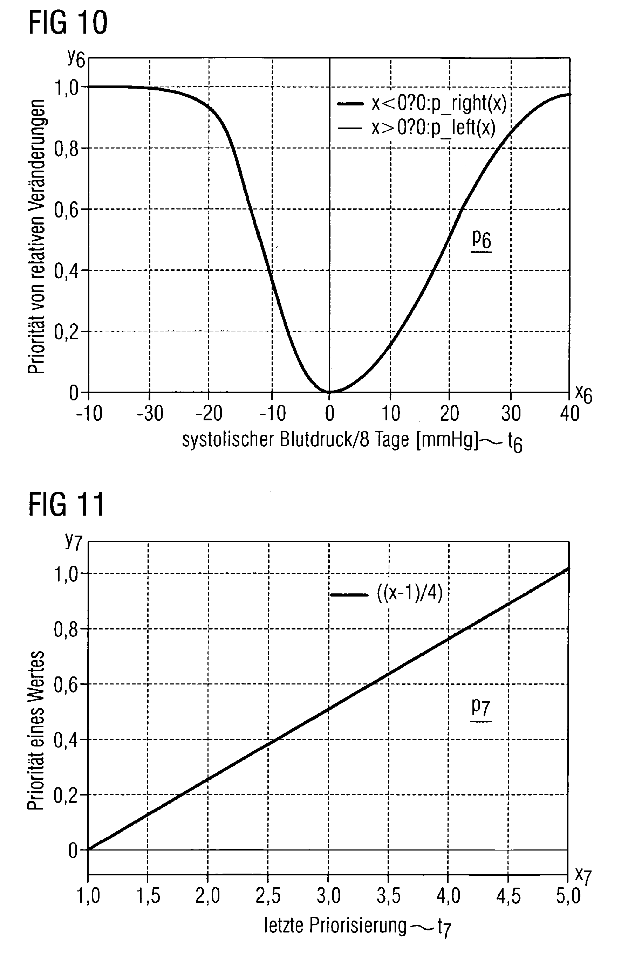

Eine Formel (6) entsprechende graphische Darstellung ist in Figur 10 gezeigt. In Tabelle 8 sind mögliche Prioritäten angegeben, welche durch einen entsprechend ermittelten Trend eines systolischen Blutdrucks über 8 Tage des zumindest einen Patienten gemäß der Formel (6) ermittelt werden können.

**Tabelle 8**

| Veränderung in mmHg | Priorität | Veränderung in mmHg | Priorität |
|---|---|---|---|
| -40,000 | 1,000 | 1,000 | 0,001 |
| -30,000 | 0,999 | 2,000 | 0,005 |
| -20,000 | 0,939 | 3,000 | 0,012 |
| -15,000 | 0,750 | 4,000 | 0,022 |
| -1 0,000 | 0,408 | 5,000 | 0,034 |
| -5,000 | 0,108 | 10,000 | 0,137 |
| -4,000 | 0,069 | 15,000 | 0,300 |
| -3,000 | 0,039 | 20,000 | 0,501 |
| -2,000 | 0,017 | 30,000 | 0,845 |
| -1,000 | 0,004 | 40,000 | 0,976 |
| 0,000 | 0,000 | - | - |

Beispielsweise weist entsprechend der Basisfunktion $p_6$ ein Trend von $x_6 = 4000$ eines systolischen Blutdrucks zumindest eines Patienten, welche innerhalb eines Zeitintervalls von 8 Tagen aus entsprechenden erfaßten Werten eines systolischen Blutdrucks des zumindest einen Patienten ermittelt wurde, eine Priorität $y_6 = 0,022$ auf. Damit ist eine geringer Anstieg eines systolischen Blutdrucks des zumindest einen Patienten innerhalb von 8 Tagen als unkritisch zu betrachten und erhält somit eine niedrige Priorität, während ein jeder weiterer Anstieg eines systolischen Blutdrucks eine exponentielle Verschlechterung bedeuten bzw. entsprechen kann, so daß die entsprechende Priorität entsprechend exponentiell steigt.

Eine Basisfunktion zur Ermittlung einer Priorität einer letzten Priorisierung zumindest eines Patienten ist bevorzugt durch eine in Figur 11 graphisch dargestellte Funktion definiert. In Tabelle 9 sind mögliche Prioritäten angegeben, welche durch eine entsprechende ermittelte letzte Priorisierung zumindest eines Patienten gemäß der in Figur 11 dargestellten Funktion ermittelt werden können.

**Tabelle 9**

| Letzte Priorisierung | Priorität |
|---|---|
| 1 | 0 |
| 2 | 0,25 |
| 3 | 0,5 |
| 4 | 0,75 |
| 5 | 1 |

Beispielsweise weist entsprechend der Basisfunktion $p_7$ einer letzten Priorisierung mit einem erfaßten Wert von $x_7 = 2$ einer letzten Priorisierung zumindest eines Patienten eine Priorität $y_7 = 0,25$ auf. Damit gewinnt ein Anstieg einer letzten Priorisierung des zumindest einen Patienten gleichmäßig an Bedeutung bezüglich einer Bearbeitungsreihenfolge des zumindest einen Patienten und schiebt den Patienten entsprechend nach vorne. Entsprechend steigt eine Priorität $y_7$ einer letzten Priorisierung linear in eines Größe eines zugeordneten Wertes.

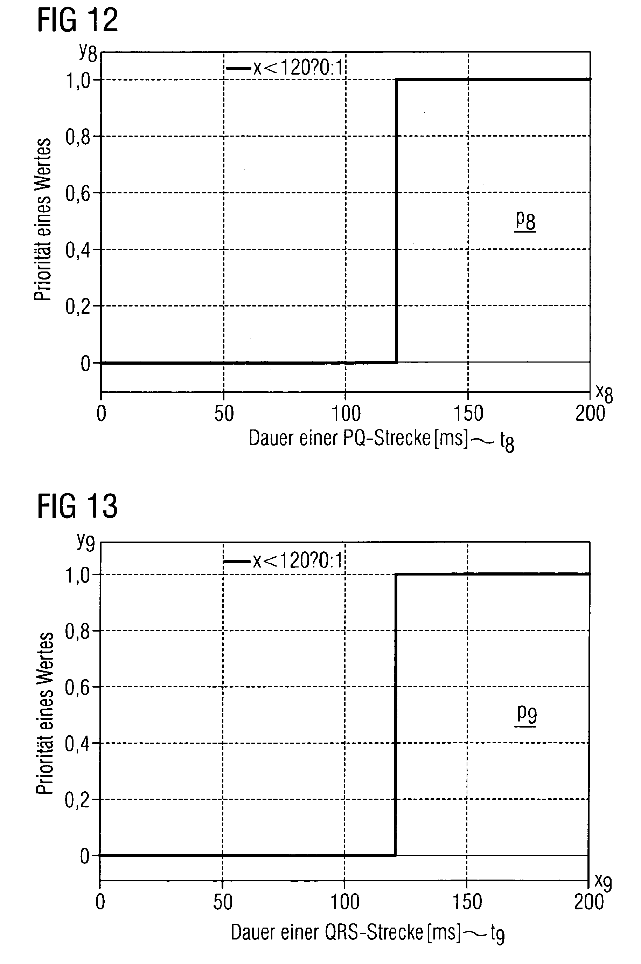

Eine Basisfunktion zur Ermittlung einer Priorität eines Wertes einer in einem EKG ermittelten Dauer einer PQ-Strecke zumindest eines Patienten ist bevorzugt durch eine in Figur 12 graphisch dargestellte Funktion definiert. In Tabelle 10 sind mögliche Prioritäten angegeben, welche durch eine entsprechende ermittelte Dauer einer PQ-Strecke zumindest eines Patienten gemäß der in Figur 12 dargestellten Funktion ermittelt werden können.

**Tabelle 10**

| PQ Dauer in ms | Priorität |
|---|---|
| <120 | 0 |
| >=120 | 1 |

Beispielsweise weist entsprechend der Basisfunktion $p_8$ einer Dauer einer PQ-Strecke mit einem erfaßten Wert von $x_8 = 110$ einer Dauer einer PQ-Strecke zumindest eines Patienten eine Priorität $y_8 = 0$ auf. Damit ist bis zu einem bestimmten Schwellenwert eine Priorität eines Wertes einer Dauer einer PQ-Strecke ganz niedrig, nämlich 0. Übersteigt eine erfaßter Werte einer Dauer einer PQ-Strecke zumindest eines Patienten einen bestimmten Schwellenwert, so gewinnt dieser Wert extrem an Beteutung hinsichtlich einer Position des zumindest einen Patienten in einer Bearbeitungsreihenfolge. Entsprechend ist eine Priorität für einen erfaßten Wert einer Dauer einer PQ-Strecke welcher über dem Schwellenwert liegt 1.

Eine Basisfunktion zur Ermittlung einer Priorität eines Wertes einer in einem EKG ermittelten Dauer einer QRS-Strecke zumindest eines Patienten ist bevorzugt durch eine in Figur 13 graphisch dargestellte Funktion definiert. In Tabelle 11 sind mögliche Prioritäten angegeben, welche durch eine entsprechende ermittelte Dauer einer QRS-Strecke zumindest eines Patienten gemäß der in Figur 13 dargestellten Funktion ermittelt werden können.

**Tabelle 11**

| QRS Dauer in ms | Priorität |
|---|---|
| <120 | 0 |
| >=120 | 1 |

Beispielsweise weist entsprechend der Basisfunktion $p_9$ einer Dauer einer QRS-Strecke mit einem erfaßten Wert von $x_9 = 110$ einer Dauer einer PQ-Strecke zumindest eines Patienten eine Priorität $y_9 = 0$ auf. Damit ist bis zu einem bestimmten Schwellenwert eine Priorität eines Wertes einer Dauer einer QRS-Strecke ganz niedrig, nämlich 0. Übersteigt eine erfaßter Werte einer Dauer einer QRS-Strecke zumindest eines Patienten einen bestimmten Schwellenwert, so gewinnt dieser Wert extrem an Bedeutung hinsichtlich einer Position des zumindest einen Patienten in einer Bearbeitungsreihenfolge. Entsprechend ist eine Priorität für einen erfaßten Wert einer Dauer einer QRS-Strecke welcher über dem Schwellenwert liegt 1.

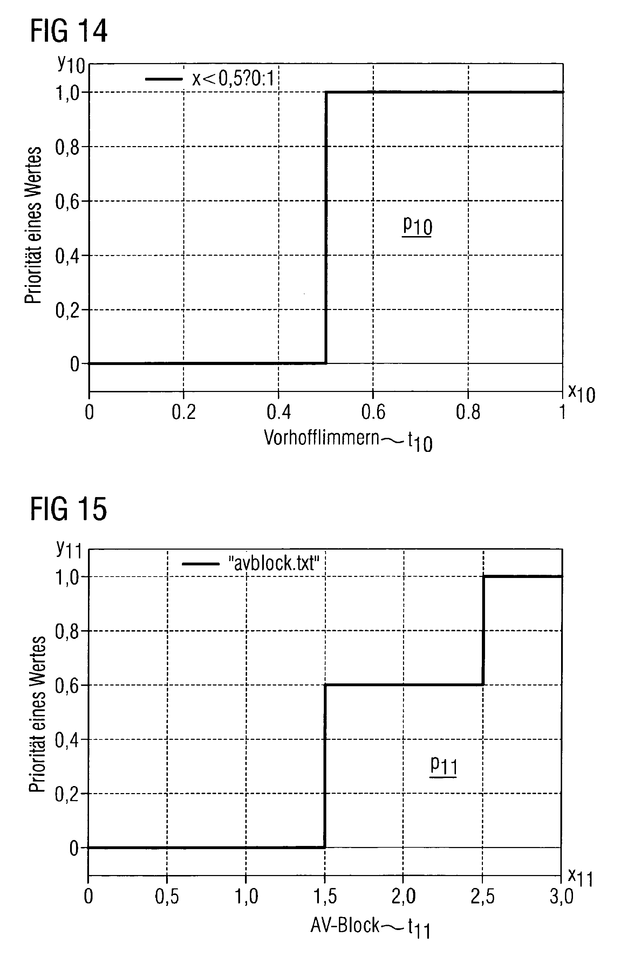

Eine Basisfunktion zur Ermittlung einer Priorität eines in einem EKG erfaßten Vorhofflimmerns zumindest eines Patienten ist bevorzugt durch eine in Figur 14 graphisch dargestellte Funktion definiert. In Tabelle 12 sind mögliche Prioritäten angegeben, welche durch ein entsprechend erfaßtes Vorhofflimmern zumindest eines Patienten gemäß der in Figur 14 dargestellten Funktion ermittelt werden können.

**Tabelle 12**

| Vorhofflimmern | Priorität |
|---|---|
| (Nicht Vorhanden) | 0 |
| (Vorhanden) | 1 |

Beispielsweise weist entsprechend der Basisfunktion $p_{10}$ für ein mögliches Vorhofflimmerns zumindest eines Patienten eine Priorität $y_{10} = 0$ auf, wenn kein Vorhofflimmern des zumindest einen Patienten erfaßt wurde. Eine Priorität der Basisfunktion für ein mögliches Vorhofflimmerns zumindest eines Patienten hat eine Priorität 1, wenn ein Vorhofflimmern des zumindest einen Patienten erfaßt wurde. Entsprechend rückt der zumindest eine Patient aufgrund der höheren Priorität in einer Bearbeitungsreihenfolge nach vorne.

Eine Basisfunktion zur Ermittlung einer Priorität eines in einem EKG erfaßten AV-Blocks zumindest eines Patienten ist bevorzugt durch eine in Figur 15 graphisch dargestellte Funktion definiert. In Tabelle 13 sind mögliche Prioritäten angegeben, welche durch ein entsprechend erfaßter AV-Block zumindest eines Patienten gemäß der in Figur 15 dargestellten Funktion ermittelt werden können.

**Tabelle 13**

| AV-Block, | Priorität |
|---|---|
| 0 (Nicht Vorhanden) | 0 |
| 1 (1.ten Grades) | 0 |
| 2 (2.ten Grades) | 0,6 |
| 3 (3.ten Grades) | 1 |

Beispielsweise weist entsprechend der Basisfunktion $P_{11}$ für einen AV-Block zumindest eines Patienten eine Priorität $y_{10} = 0$ auf, wenn kein AV-Block oder ein AV-Block ersten Grades des zumindest einen Patienten erfaßt wurde. Eine Priorität der Basisfunktion für einen AV-Block zumindest eines Patienten steigt sprunghaft auf den Wert $y_{11} = 0,6$, wenn ein AV-Block zweiten Grades des zumindest einen Patienten erfaßt wurde. Bei einem erfaßten AV-Block dritten Grades wird eine Priorität $y_{11} = 1$ ermittelt. Entsprechend rückt der zumindest eine Patient aufgrund der höheren Priorität in einer Bearbeitungsreihenfolge nach vorne.

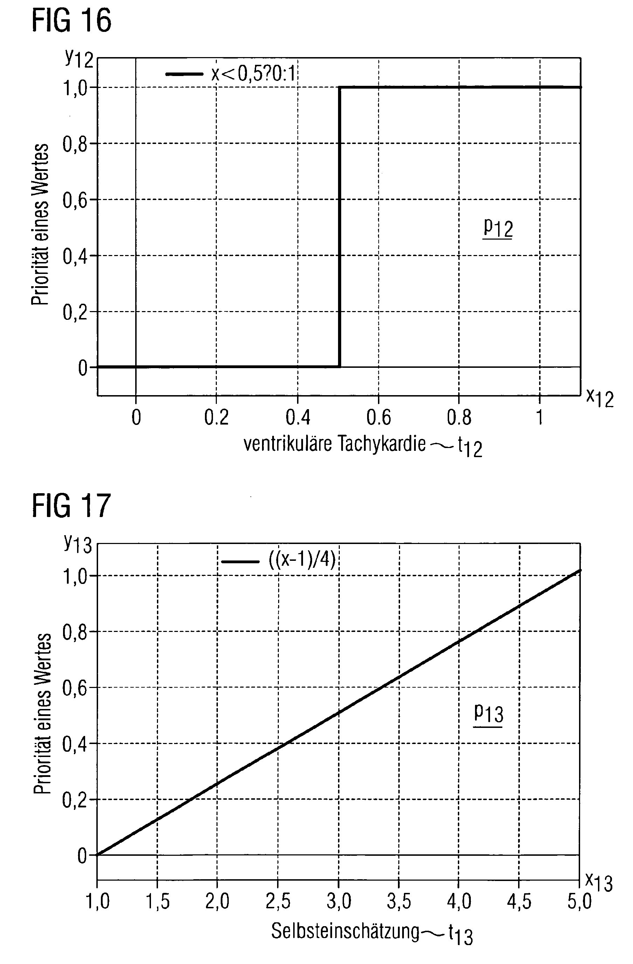

Eine Basisfunktion zur Ermittlung einer Priorität einer in einem EKG erfaßten ventrikulären Tachykardie zumindest eines Patienten ist bevorzugt durch eine in Figur 16 graphisch dargestellte Funktion definiert. In Tabelle 14 sind mögliche Prioritäten angegeben, welche durch eine entsprechend erfaßte ventrikuläre Tachykardie zumindest eines Patienten gemäß der in Figur 16 dargestellten Funktion ermittelt werden können.

**Tabelle 14**

| Ventrikuläre Tachykardie | Priorität |
|---|---|
| 0 (Nicht Vorhanden) | 0 |
| 1 (Vorhanden) | 1 |

Beispielsweise weist entsprechend der Basisfunktion $P_{12}$ für eine mögliche ventrikuläre Tachykardie zumindest eines Patienten eine Priorität $y_{12} = 0$ auf, wenn keine ventrikuläre Tachykardie des zumindest einen Patienten erfaßt wurde. Eine Priorität der Basisfunktion für eine mögliche ventrikuläre Tachykardie zumindest eines Patienten hat eine Priorität 1, wenn eine ventrikuläre Tachykardie des zumindest einen Patienten erfaßt wurde. Entsprechend rückt der zumindest eine Patient aufgrund der höheren Priorität in einer Bearbeitungsreihenfolge nach vorne.

Eine Basisfunktion zur Ermittlung einer Priorität einer Selbsteinschätzung zumindest eines Patienten ist bevorzugt durch eine in Figur 17 graphisch dargestellte Funktion definiert. In Tabelle 15 sind mögliche Prioritäten angegeben, welche durch eine entsprechende ermittelte Selbsteinschätzung zumindest eines Patienten gemäß der in Figur 17 dargestellten Funktion ermittelt werden können.

**Tabelle 15**

| Selbsteinschätzung | Priorität |
|---|---|
| 1 | 0 |
| 2 | 0,25 |
| 3 | 0,5 |
| 4 | 0,75 |
| 5 | 1 |

Beispielsweise weist entsprechend der Basisfunktion $p_{13}$ einer Selbsteinschätzung mit einem erfaßten Wert von $x_{13} = 2$ einer Selbsteinschätzung zumindest eines Patienten eine Priorität $y_{13} = 0,25$ auf. Damit gewinnt ein Anstieg einer Selbsteinschätzung des zumindest einen Patienten gleichmäßig an Bedeutung bezüglich einer Bearbeitungsreihenfolge des zumindest einen Patienten und schiebt den Patienten entsprechend aufgrund der höheren Priorität nach vorne. Entsprechend steigt eine Priorität $y_{13}$ einer Selbsteinschätzung linear in eines Größe eines zugeordneten Wertes.

Es soll so zu verstehen sein, daß die zuvor beschriebenen beispielhaften Basisfunktionen und entsprechenden Formeln eine beispielhafte Ausführungsform beschreiben. Entsprechend können in einer bevorzugten Ausführungsform die beispielhaften Basisfunktionen als Eingabe zur Ermittlung von Testergebnissen verwendet werden, um die verwendeten Basisfunktionen entsprechend zu validieren und/oder in nachfolgenden Testreihen entsprechend zu korrigieren.

Weiterhin bevorzugt erhält jede Basisfunktion des Satzes von Prioritätsfunktionen einen Gewichtungsfaktor. In Tabelle 16 sind entsprechend bevorzugte Gewichtungen eines Satzes an bevorzugten Basisfunktionen beschrieben:

**Tabelle 16**

| Nr. | Parameter | Gewichtung |
|---|---|---|
| 1 | Auftreten ventrikuläre Tachykardie | muß immer oben liegen |
| 2 | Auftreten Vorhofflimmern | muß immer oben liegen |
| 3 | Auftreten AV-Block 2. Grades | muß immer oben liegen |
| 4 | Priorisierung des Vortags | |
| 5 | Körpergewicht über 24Stunden | |
| 6 | Körpergewicht über 8 Tage | 24 zu 8 gleich |
| 7 | Systolischer Blutdruck über 24 Stunden | 24 zu 8 gleich |
| 8 | Systolisch Blutdruck über 8 Tage | |
| 9 | Selbsteinschätzung | |
| 10 | Herzfrequenz über 24 Stunden | 24 zu 8 gleich |
| 11 | Herzfrequenz über 8 Tage | |
| 12 | EKG QTc Dauer | PQ=QRS=QTc PQ ist doppelt so relevant |
| 13 | EKG PQ Dauer | |
| 14 | EKG ORS Dauer | |

Die bevorzugte Gewichtung jeder Basisfunktion des Satzes ist gemäß Tabelle 16 in eine Reihenfolge gebracht. Weiterhin bevorzugt hat (entsprechend Tabelle 16) beispielsweise ein Erfassen eines Auftretens eines ventrikulären Tachykardie zumindest eines Patienten eine höhere Gewichtung als ein Erfassen eines Auftretens eines Vorhofflimmerns zumindest eines Patienten. Entsprechend bevorzugt hat ein durch ein EKG erfaßter Wert einer QRS Dauer eine geringere Gewichtung relativ zu jeder anderen Basisfunktion des Satzes von Basisfunktionen. Weiterhin bevorzugt sind die Basisfunktionen nicht abhängig voneinander gewichtet. Entsprechend werden Abhängigkeiten zwischen einem oder mehreren entsprechenden Eingabewerte nicht berücksichtigt.

Bevorzugt werden die gewichteten Funktionen durch die Formel (7) addiert und normalisiert, um eine Priorisierung zu ermitteln. Eine Priorität einer gewichteten Funktion ist auf einen Wertebereich eines geschlossenen Intervalls zwischen 0 und 1 [0..1] normalisiert.

$$p = \frac{1}{\sum_{i=1}^{n} \omega_i} \sum_{i=1}^{n} \omega_i \cdot p_i$$

(7)

Entsprechend kann durch die gewichtete Additionen der Basisfunktionen, welche bevorzugt kontinuierlich sind, erreicht werden, daß die durch das Priorisierungsverfahren ermittelte Priorität für zumindest einen Satz von Eingabewerten zumindest eines Patienten keine Definitionslücken oder Unstetigkeiten aufweist.

Ein weiter Aspekt der vorliegenden Erfindung betrifft einen Fall, in dem nicht für jede der bevorzugten Basisfunktionen ein Eingabewert vorhanden ist. Ein Eingabewert kann beispielsweise für eine oder mehrere der bevorzugten Größen Körpergewicht, Herzfrequenz und/oder systolischer Blutdruck zumindest eines Patienten fehlen, wenn kein entsprechender Trend innerhalb eines gegebenen Zeitintervalls ermittelt werden kann. Ein Trend kann bevorzugt nur dann ermittelt werden, wenn mindestens zwei der Größe entsprechende Werte zumindest eines Patienten innerhalb eines gegebenen Zeitintervalls erfaßt werden konnten. Kann kein Trend von einer zumindest einer Größe entsprechenden Werten ermittelt werden, so hat die der Größe entsprechende Basisfunktion eine Gewichtung 0. Weiterhin bevorzugt hat eine Basisfunktion, welche als Eingabewert einen einer durch ein EKG erfaßten Werte entsprechenden Größe aufweist, bevorzugt ein durch ein EKG erfaßtes Vorhofflimmern, ein durch ein EKG erfaßter AV-Block, eine durch ein EKG erfaßte ventrikuläre Tachykardie, ein Wert einer durch ein EKG erfaßten Dauer einer PQ-Strecke, einen Wert einer durch ein EKG erfaßten Dauer einer QRS-Strecke und/oder einen Wert einer durch ein EKG erfaßten Dauer einer QTc-Strecke aufweist, eine Gewichtung 1, wenn ein entsprechender Eingabewert nicht vorliegt. Entsprechend kann ein schlechtes und/oder korrumpiertes EKG Signal in einer Ermittlung einer Priorität berücksichtigt werden.

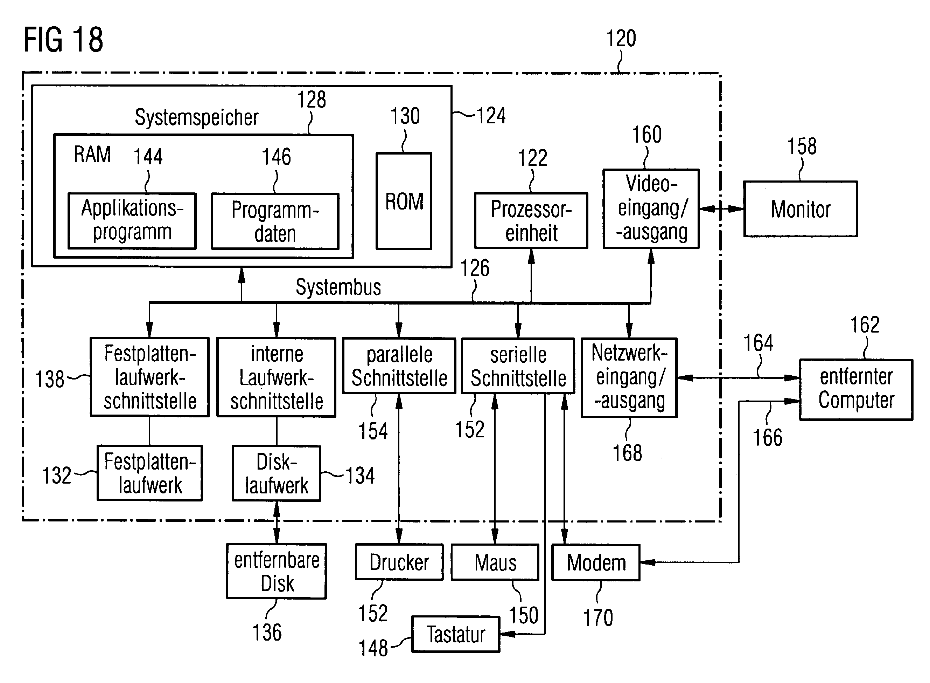

Bezugnehmend auf Figur 18 wird ein beispielhaftes System zum Implementieren der Erfindung beschrieben. Ein beispielhaftes System umfaßt eine universelle Rechnereinrichtung in der Form einer herkömmlichen Rechnerumgebung 120 z.B. ein "personal computer" (PC) 120, mit einer Prozessoreinheit 122, einem Systemspeicher 124 und einem Systembus 126, welcher eine Vielzahl von Systemkomponenten, unter anderem den Systemspeicher 124 und die Prozessoreinheit 122 verbindet. Die Prozessoreinheit 122 kann arithmetische, logische und/oder Kontrolloperationen durchführen, indem auf den Systemspeicher 124 zugegriffen wird. Der Systemspeicher 124 kann Informationen und/oder Instruktionen zur Verwendung in Kombination mit der Prozessoreinheit 122 speichern. Der Systemspeicher 124 kann flüchtige und nichtflüchtige Speicher, beispielsweise "random access memory" (RAM) 128 und "Nur-Lesespeicher" (ROM) 130 beinhalten. Ein Grund-Eingabe-Ausgabe-System (BIOS), das die grundlegenden Routinen enthält, welche helfen, Informationen zwischen den Elementen innerhalb des PCs 120, beispielsweise während des Hochfahrens, zu transferieren, kann in dem ROM 130 gespeichert sein. Der Systembus 126 kann eine von vielen Busstrukturen sein, unter anderem ein Speicherbus oder ein Speichercontroller, ein peripherer Bus und ein lokaler Bus, welcher eine bestimmte Busarchitektur aus einer Vielzahl von Busarchitekturen verwendet.

Der PC 120 kann weiterhin ein Festplattenlaufwerk 132 zum Lesen oder Schreiben einer Festplatte (nicht gezeigt) aufweisen und ein externes Disklaufwerk 134 zum Lesen oder Schreiben einer entfernbaren Disk 136 bzw. eines entfernbaren Datenträgers. Die entfernbare Disk kann eine magnetische Disk bzw. eine magnetische Diskette für ein magnetisches Disklaufwerk bzw. Diskettenlaufwerk oder eine optische Diskette wie z.B. eine CD-ROM für ein optisches Disklaufwerk sein. Das Festplattenlaufwerk 132 und das externe Disklaufwerk 134 sind jeweils mit dem Systembus 126 über eine Festplattenlaufwerkschnittstelle 138 und eine externe Disklaufwerkschnittstelle 140 verbunden. Die Laufwerke und die zugeordneten computerlesbaren Medien stellen einen nichtflüchtigen Speicher computerlesbarer Instruktionen, Datenstrukturen, Programm-Modulen und anderer Daten für den PC 120 zur Verfügung. Die Datenstrukturen können die relevanten Daten zum Implementieren eines wie oben beschriebenen Verfahrens aufweisen. Obwohl die beispielshaft beschriebene Umgebung eine Festplatte (nicht gezeigt) und eine externe Disk 142 verwendet, ist für den Fachmann offensichtlich, daß andere Typen computerlesbarer Medien, welche computerzugreifbare Daten speichern können, in der beispielhaften Arbeitsumgebung verwendet werden können, wie z.B. magnetische Kassetten, Flash-Memory Karten, digitale Videodisketten, Random-Access-Speicher, Nur-Lesespeicher, usw..

Eine Vielzahl von Programm-Modulen, insbesondere ein Betriebssystem (nicht gezeigt) ein oder mehrere Applikationsprogramme 144, oder Programm-Module (nicht gezeigt) und Programmdaten 146, können auf der Festplatte, der externen Disk 142, dem ROM 130 oder dem RAM 128 gespeichert werden. Die Applikationsprogramme können zumindest einen Teil der Funktionalität, wie in Figur 10 gezeigt, umfassen.

Ein Benutzer kann Kommandos und Information, wie oben beschrieben, in den PC 120 anhand von Eingabevorrichtungen, wie z.B. einer Tastatur bzw. eines Keyboards 148 und einer Computermaus bzw. einem Trackball 150 eingeben. Andere Eingabevorrichtungen (nicht gezeigt) können ein Mikrofon und/andere Sensoren, einen Joystick, ein Spielpolster bzw. -kissen, einen Scanner oder ähnliches umfassen. Diese oder andere Eingabevorrichtungen können mit der Prozessoreinheit 122 anhand einer seriellen Schnittstelle 152 verbunden sein, welche mit dem System 126 gekoppelt ist, oder können anhand anderer Schnittstellen, wie z.B. einer parallelen Schnittstelle 154, eines Spieleports oder eines universellen seriellen Busses (USB) verbunden sein. Weiterhin kann Information mit einem Drucker 156 gedruckt werden. Der Drucker 156 und andere parallele Eingabe/Ausgabevorrichtungen können mit der Prozessoreinheit 122 durch die parallele Schnittstelle 154 verbunden sein. Ein Monitor 158 oder andere Arten von Anzeigevorrichtung (en) ist/sind mit dem Systembus 126 mittels einer Schnittstelle, wie z.B. eines Videoeingang/-ausgangs 160 verbunden. Zusätzlich zu dem Monitor kann die Rechnerumgebung 120 andere periphere Ausgabevorrichtungen (nicht gezeigt) wie z.B. Lautsprecher oder akustische Ausgänge umfassen.

Die Rechnerumgebung 120 kann mit anderen elektronischen Vorrichtungen z.B. einem Computer, einem Schnurtelefon, einem schnurlosen Telefon, einem persönlichen digitalen Assistenten (PDA), einem Fernseher oder ähnlichem kommunizieren. Um zu kommunizieren, kann die Rechnerumgebung 120 in einer vernetzten Umgebung arbeiten, wobei Verbindungen zu einem oder mehreren elektronischen Vorrichtungen verwendet werden. Figur 10 stellt die mit einem "remote computer" bzw. entfernten Computer 162 vernetzte Rechnerumgebung dar. Der entfernte Computer 162 kann eine andere Rechnerumgebung, wie z.B. ein Server, ein Router, ein Netzwerk-PC, eine gleichwertige bzw. "peer" Vorrichtung oder andere gewöhnliche Netzwerkknoten sein und kann viele oder alle der hinsichtlich der Rechnerumgebung 120 oben beschriebenen Elemente umfassen. Die logischen Verbindungen, wie sie in Figur 10 dargestellt sind, umfassen ein "local area network" (LAN) 164 und ein "wide are network" (WAN) 166. Solche Netzwerkumgebungen sind alltäglich in Büros, firmenweiten Computernetzwerken, Intranetzen und dem Internet.

Wenn eine Rechnerumgebung 120 in einer LAN-Netzwerkumgebung verwendet wird, kann die Rechnerumgebung 120 mit dem LAN 164 durch einen Netzwerkeingang/-ausgang 168 verbunden sein. Wenn die Rechnerumgebung 120 in einer WAN-Netzwerkumgebung verwendet wird, kann die Rechnerumgebung 120 ein Modem 170 oder andere Mittel zum Herstellen einer Kommunikation über das WAN 166 umfassen. Das Modem 170, welches intern und extern bezüglich der Rechnerumgebung 120 sein kann, ist mit dem Systembus 126 mittels der seriellen Schnittstelle 152 verbunden. In der Netzwerkumgebung können Programm-Module, welche relativ zu der Rechnerumgebung 120 dargestellt sind, oder Abschnitte davon in einer entfernten Speichereinrichtung gespeichert sein, welche an oder von einem entfernten Computer 162 zugreifbar bzw. systemeigen sind. Weiterhin können andere Daten, welche für das oben beschriebene Verfahren bzw. System relevant sind, auf oder von dem entfernten Computer 162 zugreifbar vorliegen.

## Bezugszeichenliste

| | |
|---|---|
| 1 | Sensor |
| 2 | mobiler Assistent |
| 3 | Service Center |
| 4 | Service Bus |
| 5 | telemedizinischer Arbeitsplatz |
| 41 | Prorisierungsverfahren |
| 42 | elektronische Patientenakte |
| 43 | Aufgabengenerator |
| 120 | Rechnerumgebung |
| 122 | Prozessoreinheit |
| 124 | Systemspeicher |
| 126 | Systembus |
| 128 | random access memory (RAM) |
| 130 | Nur-Lesespeicher (ROM) |
| 132 | Festplattenlaufwerk |
| 134 | Disklaufwerk |
| 136 | entfernbare Disk |
| 138 | Festplattenlaufwerkschnittstelle |
| 140 | Disklaufwerkschnittstelle |
| 142 | externe Disk |

| 144 | Applikationsprogramm |
| --- | --- |
| 146 | Programmdaten |
| 148 | Tastatur |
| 150 | Computermaus/Trackball |
| 152 | serielle Schnittstelle |
| 154 | parallele Schnittstelle |
| 156 | Drucker |
| 158 | Monitor |
| 160 | Videoeingang/ -ausgang |
| 162 | entfernter Computer |
| 164 | local area network (LAN) |
| 166 | wide area network (WAN) |
| 168 | Netzwerkeingang/ -ausgang |

**Patentansprüche**

1. Computer-implementiertes Verfahren zu einer automatischen Priorisierung von medizischen Betreuungen von zumindest einem Patienten, die Schritte umfassend:

- Erfassen von einem oder mehreren Werten des zumindest einen Patienten (S1);
- Bestimmen zumindest eines Trends des zumindest einen Patienten zu einer entsprechenden Größe (S3);
- Bereitstellen eines Satzes von gewichteten Funktionen ($p_1,...,p_m$) und eines Satzes von medizinischen Größen ($t_1,...,t_m$), wobei jeweils zumindest eine gewichtete Funktion ($p_i$) des Satzes von gewichteten Funktionen jeweils zumindest einer Größe ($t_i$) des Satzes von medizinischen Größen zugeordnet ist;
- Anwenden des Satzes von gewichteten Funktionen auf einen oder mehrere Werte des zumindest einen Patienten und/oder auf zumindest einen Trend des zumindest einen Patienten, wobei jeweils zumindest eine gewichtete Funktion ($p_i$) des Satzes von gewichteten Funktionen auf zumindest einen der zumindest einen zugeordneten Größe ($t_i$) entsprechenden Wert ($x_i$) und/oder auf zumindest einen der zumindest einen zugeordneten Größe ($t_i$) entsprechenden Trend ($x_i$) angewendet wird (S41);
- Addieren und Normalisieren der jeweiligen gewichteten Funktionen ($p_1,...,p_m$) zum Erhalt einer Priorisierung (S42 und S5); und
- Ausgeben einer Betreuungsreihenfolge für den zumindest einen Patienten, welche der Priorisierung entspricht.

2. Computer-implementiertes Verfahren nach Anspruch 1, der Schritt des Erfassens von einem oder mehreren Werten zumindest die Schritte umfassend:

- Erfassen eines Wertes eines Körpergewichts des zumindest einen Patienten; und/oder
- Erfassen eines Wertes einer Herzfrequenz des zumindest einen Patienten; und/oder
- Erfassen eines Wertes eines systolischen Blutdrucks des zumindest einen Patienten; und/oder
- Erfassen einer zuvor manuell gesetzten Priorisierung des zumindest einen Patienten; und/oder
- Erfassen eines Wertes einer Dauer einer PQ-Strecke des zumindest einen Patienten; und/oder
- Erfassen eines Wertes einer Dauer einer QRS-Strecke des zumindest einen Patienten; und/oder
- Erfassen eines Wertes einer Dauer einer QTc-Strecke des zumindest einen Patienten; und/oder
- Erfassen einer Selbsteinschätzung des zumindest einen Patienten; und/oder
- Erfassen eines in einem EKG erfaßten Vorhofflimmers des zumindest einen Patienten; und/oder
- Erfassen eines in einem EKG erfaßten AV Blockes ersten, zweiten oder dritten Grades des zumindest einen Patienten; und/oder
- Erfassen eines in einem EKG erfaßten ventrikuläre Tachykardie des zumindest einen Patienten.

3. Computer-implementiertes Verfahren nach Anspruch 1 oder 2, wobei der Schritt des Erfassens von einem oder mehreren Werten des zumindest einen Patienten durch zumindest ein geeignetes medizinisches Gerät erfolgt.

4. Computer-implementiertes Verfahren nach einem oder mehreren der vorangegangenen Ansprüche, wobei der Schritt des Erfassens von einem oder mehreren Werten des zumindest einen Patienten durch zumindest einen Assessmentbogen erfolgt.

5. Computer-implemetiertes Verfahren nach einem oder mehreren der vorangegangenen Ansprüche, der Schritt des Bestimmens zumindest eines Trends des zumindest einen Patienten zu einer entsprechenden Größe, den Schritt umfassend:

- Erfassen von zumindest einem einer Größe entsprechenden Wert innerhalb eines Zeitintervalls.

6. Computer-implementiertes Verfahren nach Anspruch 5, das Zeitintervall 24 Stunden und/oder 8 Tagen beträgt.

7. Computer-implementiertes Verfahren nach Anspruch 5 oder 6, wobei die Größe ein Körpergewicht der zumindest einen Patienten ist.

8. Computer-implementiertes Verfahren nach Anspruch 5 oder 6, wobei die Größe eine Herzfrequenz des zumindest einen Patienten ist.

9. Computer-implementiertes Verfahren nach Anspruch 5 oder 6, wobei die Größe ein systolischer Blutdruck des zumindest einen Patienten ist.

10. Computer-implementiertes Verfahren nach einem oder mehreren der Ansprüche 5 bis 9, der Schritt des Bestimmen zumindest eines Trends des zumindest einen Patienten zu einer entsprechenden Größe, die Schritte umfassend:

- Berechnen des Trends durch eine Steigung einer Geraden durch zwei Punkte, wobei die zwei Punkte zumindest zwei Werte der Menge von erfaßten Werten innerhalb des Zeitintervalls umfaßt; und/oder
- Berechnen des Trends durch eine Steigung einer Geraden, welche in einem "Least-Square-Fitting" Verfahren in die Menge von Werten gelegt wird, welche innerhalb des Zeitintervalls erfaßt sind.

11. Computer-implementiertes Verfahren nach einem oder mehreren der vorangehenden Ansprüche, wobei der Schritt des Addierens und Normalisierens der jeweiligen gewichteten Funktionen ein Normalisieren auf einen Wertebereich [0..1] umfaßt.

12. System zu einer automatischen Priorisierung von medizischen Betreuungen von zumindest einem Patienten, umfassend:

- einen Server (4), welcher ausgelegt ist, zumindest einen Wert des zumindest einen Patienten zu erfassen, umfassend:
- eine Datenverwaltungseinrichtung (42), welche ausgelegt ist, den zumindest einen Wert dem zumindest einen Patienten zuzuordnen und zu speichern;
- eine Priorisierungseinrichtung (41), welche ausgelegt ist, ein Verfahren nach einem oder mehreren der Ansprüche 1 bis 9 durchzuführen; und
- ein Generator (43), welcher ausgelegt ist, aus zumindest einer durch die Priorisierungseinrichtung bestimmten priorisierten Aufgabe eine Reihenfolge zu generieren; und
- einen Client (5), welcher ausgelegt ist, die Reihenfolge auszugeben und anzuzeigen.

13. Computerprogrammprodukt, insbesondere auf einem computerlesbaren Medium gespeichert oder als Signal verwirklicht, welches, wenn geladen in den Speicher eines Computers und ausgeführt von einem Computer, bewirkt, daß der Computer ein Verfahren nach einem oder mehreren der Ansprüche 1 bis 9 durchführt.

# FIG 1

**Aclimon-Telehome-Network**

1 Sensor — 2 MMA — 3 ASC — 4 TSB — **ICW-Telemonitoring platform** — 5 TMA

MMA - Mobiler medizinischer Assistent (PDA)
ASC - Aclimon Service Center (Server)
TSB - Telemedizinischer Service Bus (Server)
TMA - Telemedizinischer Arbeitsplatz (Client)

EP 1 936 522 A1

# FIG 2

**ASC** — 3

**4** — Telemedizinischer Service Bus
- Aufgaben Priorisierungs-algorithmus — 41
- Elektronische Patientenakte — 42
- Aufgaben-generator — 43

**TMA** — 5

EP 1 936 522 A1

## FIG 3

$e_1{:}t_1,..e_n{:}t_n$ — S1

speichern — S10

42
elektronische Patientenakte

für alle i aus 1...n

S2
$v(e_i)$ in T?

ja

S3
erzeuge $v(e_i)$

hole $e_{i1},...,e_{ik}$

nein

S4
generiere $x_1,...,x_m$

41

für alle j: $p_j(x_j) = y_j$ — S41

für alle j: addiere und normiere $y_j$ — S42

gebe Priorität p aus — S5

# FIG 3A

41

$x_1$ Körpergewicht/24 Std [kg] →

$x_2$ Körpergewicht/8 Tage [kg] →

$x_3$ Herzfrequenz/24 Std [bpm] →

$x_4$ Herzfrequenz/8 Tage [bpm] →

$x_5$ systolischer Blutdruck/24 Std [mmHg] →

$x_6$ systolischer Blutdruck/8 Tage [mmHg] →

$x_7$ letzte Priorisierung →

$x_8$ EKG PQ Dauer [ms] →

$x_9$ EKG QRS Dauer [ms] →

$x_{10}$ EKG QTc Dauer [ms] →

$x_{11}$ Selbsteinschätzung (1, 2, 3, 4, 5) →

$x_{12}$ Vorhofflimmern (0, 1) →

$x_{13}$ AV Block (0, 1, 2, 3 ) →

$x_{14}$ ventrikuläre Tachykardie →

Priorisierungs-algorithmus → Priorisierung y

EP 1 936 522 A1

# FIG 4

$P_i(x) = y_i$
Basisfunktion von relativen Veränderungen

Priorität von relativen Veränderungen

$P_i(-0,5) = 0,5$

$x_i = -0,5$ kg    Körpergewicht/8 Tage [kg]

# FIG 5

Priorität von relativen Veränderungen

$p_1$

Körpergewicht/24 Stunden [kg] $\sim t_1$

## FIG 6

Körpergewicht/8 Tage [kg] ～ t₂

## FIG 7

Herzfrequenz/24 Stunden [bpm] ～ t₃

## FIG 8

Herzfrequenz/8 Tage [bpm]～ t4

## FIG 9

systolischer Blutdruck/24 Stunden [mmHg]～t5

## FIG 10

Legend:
- x<0?0:p_right(x)
- x>0?0:p_left(x)

p6

y6 — Priorität von relativen Veränderungen

x6 — systolischer Blutdruck/8 Tage [mmHg] ~ t6

## FIG 11

Legend:
- ((x-1)/4)

p7

y7 — Priorität eines Wertes

x7 — letzte Priorisierung ~ t7

## FIG 12

Priorität eines Wertes

x<120?0:1

p8

Dauer einer PQ-Strecke [ms] — t8

## FIG 13

Priorität eines Wertes

x<120?0:1

p9

Dauer einer QRS-Strecke [ms] — t9

## FIG 14

x<0,5?0:1

Priorität eines Wertes

$p_{10}$

Vorhofflimmern $\sim t_{10}$

$x_{10}$

## FIG 15

"avblock.txt"

Priorität eines Wertes

$p_{11}$

AV-Block $\sim t_{11}$

$x_{11}$

## FIG 16

Priorität eines Wertes

ventrikuläre Tachykardie ⌇ t₁₂

legend: x<0,5?0:1

p₁₂

## FIG 17

Priorität eines Wertes

Selbsteinschätzung ⌇ t₁₃

legend: ((x-1)/4)

p₁₃

**FIG 18**

EP 1 936 522 A1

**EP 1 936 522 A1**

Europäisches
Patentamt

**EUROPÄISCHER RECHERCHENBERICHT**

Nummer der Anmeldung

EP 06 02 6806

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2006/116557 A1 (MOORE TIMOTHY J [US] ET AL) 1. Juni 2006 (2006-06-01) * das ganze Dokument * | 1-13 | INV. G06F19/00 |
| A | WO 99/46718 A (HEALTHWARE CORP [US]; SURWIT RICHARD S [US]; ALLEN LYLE M III [US]; CU) 16. September 1999 (1999-09-16) * Abbildungen 1-9 * * Seite 4, Zeile 15 - Zeile 20 * * Seite 16, Zeile 15 - Seite 43, Zeile 7 * | 1-13 | |
| A | DE 103 37 236 A1 (TRIUM ANALYSIS ONLINE GMBH [DE]; CANWAY TECHNOLOGY GMBH [DE]) 24. März 2005 (2005-03-24) * das ganze Dokument * | 1-13 | |

RECHERCHIERTE SACHGEBIETE (IPC)

G06F

Der vorliegende Recherchenbericht wurde für alle Patentansprüche erstellt

| Recherchenort | Abschlußdatum der Recherche | Prüfer |
|---|---|---|
| Den Haag | 1. Juni 2007 | SCHECHNER-RESOM, M |

**EP 1 936 522 A1**

**ANHANG ZUM EUROPÄISCHEN RECHERCHENBERICHT
ÜBER DIE EUROPÄISCHE PATENTANMELDUNG NR.**

EP 06 02 6806

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-06-2007

| Im Recherchenbericht angeführtes Patentdokument | Datum der Veröffentlichung | Mitglied(er) der Patentfamilie | Datum der Veröffentlichung |
|---|---|---|---|
| US 2006116557    A1 | 01-06-2006 | KEINE | |
| WO 9946718       A | 16-09-1999 | AT        239264 T<br>AU        754171 B2<br>AU       2092699 A<br>CA       2322563 A1<br>DE      69814172 D1<br>DE      69814172 T2<br>EP       1062615 A1<br>ES       2198091 T3<br>US       6024699 A<br>US       6589169 B1 | 15-05-2003<br>07-11-2002<br>27-09-1999<br>16-09-1999<br>05-06-2003<br>23-10-2003<br>27-12-2000<br>16-01-2004<br>15-02-2000<br>08-07-2003 |
| DE 10337236      A1 | 24-03-2005 | KEINE | |

EPO FORM P0461

Für nähere Einzelheiten zu diesem Anhang : siehe Amtsblatt des Europäischen Patentamts, Nr.12/82